# EUROPEAN PATENT APPLICATION

(11) **EP 4 467 568 A1**
(43) Date of publication of application: **27.11.2024**
(21) Application number: 23740107.0
(22) Date of filing: 16.01.2023
(51) Int. Cl.: C07K 16/28, A61K 39/395, A61P 35/00

(54) **ANTIBODY TARGETING CD25, AND PREPARATION METHOD THEREFOR AND USE THEREOF**

(30) Priority: 17.01.2022 CN 202210051233
(71) Applicant: Nona Biosciences (Suzhou) Co., Ltd., Suzhou, Jiangsu 215000 (CN)
(72) Inventor: PAN, Qiuming, Shanghai 201203 (CN); WANG, Chengen, Shanghai 201203 (CN); HE, Jinqiu, Shanghai 201203 (CN); LI, Yan, Shanghai 201203 (CN); WANG, Yuandong, Shanghai 201203 (CN); WANG, Yongqiang, Shanghai 201203 (CN); ZHAO, Chuchu, Shanghai 201203 (CN); PAN, Hongjie, Shanghai 201203 (CN); RONG, Yiping, Shanghai 201203 (CN); LUO, Haishan, Shanghai 201203 (CN)
(74) Representative: Murgitroyd & Company
(86) International application number: PCT/CN2023/072439
(87) International publication number: WO 2023/134766

(57) **Abstract**

Disclosed in the present invention is an antibody targeting CD25 or a variant thereof. A monoclonal antibody targeting CD25 is a naturally occurring antibody, has an activity of binding to human CD25 and cynomolgus monkey CD25, and has a stronger ADCC effect after being modified with Fc. In-vitro experiments show that the antibody has the effect of clearing Treg and lymphoma cells without blocking an IL-2 signaling pathway.

## Description

The present application claims the right of priority for the Chinese patent application No. 2022100512339 with the filling date of January 17, 2022. This Chinese patent application is incorporated herein by reference in its entirety.

### Technical Field

The present application relates to the field of biomedicine, in particular to an antibody targeting CD25 or a variant thereof, and a preparation method therefor and the use thereof.

### Background

Regulatory T cells (Tregs) are a subset of T cells that control autoimmune reactivity in the body, playing a key role in maintaining immune tolerance, autoimmunity and anti-tumor immunity in the body. Existing studies have found that the increase in tumor-infiltrating Treg cells is inversely correlated with the prognosis in a variety of tumors. In addition, the ratio of effector T cells (Teffs) to Tregs in the tumor microenvironment would affect the tumor progression and therapeutic effect. Although these studies suggest the potential value of anti-tumor treatment regimens targeting Tregs, there are few successful examples in anti-tumor therapies targeting Tregs clinically, mainly because the mechanism for the design of marker molecules that specifically target Tregs is not clear. Anti-CTLA-4 antibodies have shown the effect of clearing tumor-infiltrating Treg cells in mouse experiments by activating Fc receptors (FcRs). However, in clinical trials, the effect of anti-CTLA-4 antibodies in clearing Tregs is still unclear. Some studies have found that enhancing the affinity of anti-CTLA-4 antibodies for FcRs can improve the effect of the antibodies in clinical trials, but not all anti-CTLA-4 antibody treatments lead to the reduction of Treg cells. Therefore, there is still a need to develop specific marker molecule antibodies that target Tregs to effect Treg-targeted anti-tumor therapy.

CD25 is the α chain of interleukin-2 receptor (IL2Rα), which is constitutively and highly expressed in Treg cells, while the expression level of CD25 in Teff cells is lower. This property makes CD25 a potential target capable of specifically targeting Treg cells. However, the development of anti-CD25 antibodies is limited due to the lack of studies thereof in mouse and clinical trials. Recent studies have found that in mouse experiments, enhancing the antibody-dependent cell-mediated cytotoxicity (ADCC) by increasing the affinity of anti-CD25 antibodies for FcRs can significantly improve the anti-tumor activity of the antibodies *in vivo.* In addition, studies have found that an anti-CD25 antibody with enhanced ADCC function through Fc optimization can significantly improve the anti-tumor activity of an anti-PD-1 antibody *in vivo.* On the other hand, CD25 is expressed in Hodgkin's lymphoma and various non-Hodgkin's lymphomas, and the expression rate of CD25 is close to 100% in adult T-cell leukemia/lymphoma and hairy cell leukemia. Based on the high expression of CD25 in hematological tumors, the research on antibody-drug conjugates (ADCs) targeting CD25 has become a hot spot. ADCT-301, an existing anti-CD25 ADC drug, has shown good efficacy in clinical studies of Hodgkin's lymphoma. Based on the high expression of CD25 in Treg and lymphoma cells, the development of antibody drugs to this target can effect anti-tumor effects with enhanced ADCC function through Fc optimization. On the other hand, an ADC method can also be used for the development.

The research and development of anti-CD25 antibody drugs focused on blocking antibodies against the ligand interleukin-2 in the early stage, including monoclonal antibodies and ADCs. Recent studies have found that anti-CD25 antibodies that do not block interleukin-2 have shown a better anti-tumor activity in mouse experiments, which may be due to the expression of CD25 on effector T cells (Teffs). Blocking antibodies inhibit the interleukin-2 signaling pathway, thereby inhibiting the activity of Teffs. However, Teffs are the cells that play a main role in tumor immunity, and this property of blocking antibodies weakens the anti-tumor effect of Teffs. A non-blocking anti-CD25 antibody would avoid this problem and would have a more pronounced anti-tumor activity.

In the field with an anti-CD25 monoclonal antibody as the development strategy, many companies have been currently working on the development of the anti-CD25 interleukin-2 non-blocking antibody, with Roche's RG6292 showing the fastest progress and being in a phase I clinical trial with solid tumors as its indications. In the field with an ADC as the development strategy, there are also many companies conducting research on drugs, with ADCT-301 showing the fastest progress and being currently in a phase II clinical trial with Hodgkin's lymphoma as its indication. However, there is no highly selective anti-CD25 interleukin-2 non-blocking antibody clinically.

### Summary

The present invention provides an anti-CD25 interleukin-2 non-blocking antibody or a variant thereof that has a high affinity, high selectivity and high biological activity, has the ability to be endocytosed by cells, and is an antibody molecule suitable for the development of an ADC drug.

The first aspect of the present invention provides an antibody targeting CD25 or a variant thereof, characterized in that the antibody comprises a light chain variable region and a heavy chain variable region, wherein the antibody is selected from the following groups:
the light chain variable region comprising LCDR1, LCDR2 and LCDR3 consisting of the amino acid sequences set forth in SEQ ID NOs: 96, 111 and 133, respectively; and/or, the heavy chain variable region comprising HCDR1, HCDR2 and HCDR3 consisting of the amino acid sequences set forth in SEQ ID NOs: 20, 44 and 74, respectively; or,
the light chain variable region comprising LCDR1, LCDR2 and LCDR3 consisting of the amino acid sequences set forth in SEQ ID NOs: 104, 111 and 127, respectively; and/or, the heavy chain variable region comprising HCDR1, HCDR2 and HCDR3 consisting of the amino acid sequences set forth in SEQ ID NOs: 22, 46 and 76, respectively; or,
the light chain variable region comprising LCDR1, LCDR2 and LCDR3 consisting of the amino acid sequences set forth in SEQ ID NOs: 94, 110 and 134, respectively; and/or, the heavy chain variable region comprising HCDR1, HCDR2 and HCDR3 consisting of the amino acid sequences set forth in SEQ ID NOs: 23, 48 and 78, respectively; or,
the light chain variable region comprising LCDR1, LCDR2 and LCDR3 consisting of the amino acid sequences set forth in SEQ ID NOs: 97, 113 and 129, respectively; and/or, the heavy chain variable region comprising HCDR1, HCDR2 and HCDR3 consisting of the amino acid sequences set forth in SEQ ID NOs: 16, 40 and 70, respectively; or,
the light chain variable region comprising LCDR1, LCDR2 and LCDR3 consisting of the amino acid sequences set forth in SEQ ID NOs: 94, 116 and 135, respectively; and/or, the heavy chain variable region comprising HCDR1, HCDR2 and HCDR3 consisting of the amino acid sequences set forth in SEQ ID NOs: 23, 47 and 77, respectively; or,
the light chain variable region comprising LCDR1, LCDR2 and LCDR3 consisting of the amino acid sequences set forth in SEQ ID NOs: 95, 111 and 127, respectively; and/or, the heavy chain variable region comprising HCDR1, HCDR2 and HCDR3 consisting of the amino acid sequences set forth in SEQ ID NOs: 14, 38 and 68, respectively; or,
the light chain variable region comprising LCDR1, LCDR2 and LCDR3 consisting of the amino acid sequences set forth in SEQ ID NOs: 98, 114 and 130, respectively; and/or, the heavy chain variable region comprising HCDR1, HCDR2 and HCDR3 consisting of the amino acid sequences set forth in SEQ ID NOs: 17, 41 and 71, respectively; or,
the light chain variable region comprising LCDR1, LCDR2 and LCDR3 consisting of the amino acid sequences set forth in SEQ ID NOs: 99, 115 and 131, respectively; and/or, the heavy chain variable region comprising HCDR1, HCDR2 and HCDR3 consisting of the amino acid sequences set forth in SEQ ID NOs: 18, 42 and 72, respectively; or,
the light chain variable region comprising LCDR1, LCDR2 and LCDR3 consisting of the amino acid sequences set forth in SEQ ID NOs: 100, 113 and 132, respectively; and/or, the heavy chain variable region comprising HCDR1, HCDR2 and HCDR3 consisting of the amino acid sequences set forth in SEQ ID NOs: 14, 43 and 73, respectively; or,
the light chain variable region comprising LCDR1, LCDR2 and LCDR3 consisting of the amino acid sequences set forth in SEQ ID NOs: 103, 110 and 134, respectively; and/or, the heavy chain variable region comprising HCDR1, HCDR2 and HCDR3 consisting of the amino acid sequences set forth in SEQ ID NOs: 21, 45 and 75, respectively; or,
the light chain variable region comprising LCDR1, LCDR2 and LCDR3 consisting of the amino acid sequences set forth in SEQ ID NOs: 105, 111 and 136, respectively; and/or, the heavy chain variable region comprising HCDR1, HCDR2 and HCDR3 consisting of the amino acid sequences set forth in SEQ ID NOs: 18, 42 and 80, respectively.

It is well known to a person skilled in the art that the CDRs of an antibody can be defined in the art by a variety of methods, such as Chothia based on the three-dimensional structures of antibodies and the topology of CDR loops (Chothia et al., (1989) Nature 342: 877-883, Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (World-Wide-Web imgt.cines.fr/), and the North CDR definition based on affinity propagation clustering using a large number of crystal structures. In the present invention, the amino acid sequences of CDRs listed are shown according to the rules of the Chothia definition (the sequences in the claims of the present invention are also shown according to the rules of the Chothia definition).

In the definition, Laa-Lbb can refer to an amino acid sequence from position aa to position bb, starting from the N-terminus of an antibody light chain; and Haa-Hbb can refer to an amino acid sequence from position aa to position bb, starting from the N-terminus of an antibody heavy chain. For example, L24-L34 can refer to an amino acid sequence from position 24 to position 34 according to the Chothia coding rules, starting from the N-terminus of an antibody light chain; H26-H32 can refer to an amino acid sequence from position 26 to position 32 according to the Chothia coding rules, starting from the N-terminus of an antibody heavy chain.

It should be understood by a person skilled in the art that unless otherwise specified, the terms "CDR" and "complementarity determining region" of a given antibody or a region thereof (e.g., a variable region) should be understood to encompass a complementarity determining region as defined by any of the above-mentioned known schemes described in the present invention. Although the scope of protection as claimed in the claims of the present invention is based on the sequences shown according to the rules of the Chothia definition, corresponding amino acid sequences according to the rules of other CDR definitions shall also fall within the scope of protection of the present invention.

Therefore, when it comes to defining an antibody with specific CDR sequences defined in the present invention, the scope of the antibody also encompasses such an antibody whose variable region sequences comprise the specific CDR sequences but whose claimed CDR boundaries are different from the specific CDR boundaries defined by the present invention due to the application of a different scheme (for example, rules of various CDR definitions or their combinations).

In a certain particular embodiment, the variant results from an addition, a deletion or a substitution of 1 to 3 amino acid residues in the amino acid sequence of the antibody targeting CD25.
Preferably, the variant results from a substitution of amino acid residues at position 1 of HCDR1 and at position 3 of HCDR2 in the heavy chain variable region of the antibody, and/or, a substitution of amino acid residues at positions 10-11 of LCDR1 and at position 1 or 4 of LCDR3 in the light chain variable region of the antibody;
preferably, the amino acid sequence of the variant has at least 85% sequence identity to the original amino acid sequence, and maintains or improves the binding of the antibody to an antigen of interest; the at least 85% sequence identity is preferably at least 90% sequence identity; more preferably at least 95%, 96%, 97% or 98% sequence identity; and most preferably at least 99% sequence identity.

More preferably, the variant is selected from the following groups:
the light chain variable region comprising LCDR1, LCDR2 and LCDR3 consisting of the amino acid sequences set forth in SEQ ID NOs: 101, 113 and 129, respectively; and/or, the heavy chain variable region comprising HCDR1, HCDR2 and HCDR3 consisting of the amino acid sequences set forth in SEQ ID NOs: 19, 40 and 70, respectively; or,
the light chain variable region comprising LCDR1, LCDR2 and LCDR3 consisting of the amino acid sequences set forth in SEQ ID NOs: 101, 113 and 139, respectively; and/or, the heavy chain variable region comprising HCDR1, HCDR2 and HCDR3 consisting of the amino acid sequences set forth in SEQ ID NOs: 19, 40 and 70, respectively; or,
the light chain variable region comprising LCDR1, LCDR2 and LCDR3 consisting of the amino acid sequences set forth in SEQ ID NOs: 102, 113 and 129, respectively; and/or, the heavy chain variable region comprising HCDR1, HCDR2 and HCDR3 consisting of the amino acid sequences set forth in SEQ ID NOs: 19, 40 and 70, respectively; or,
the light chain variable region comprising LCDR1, LCDR2 and LCDR3 consisting of the amino acid sequences set forth in SEQ ID NOs: 94, 116 and 137, respectively; and/or, the heavy chain variable region comprising HCDR1, HCDR2 and HCDR3 consisting of the amino acid sequences set forth in SEQ ID NOs: 23, 50 and 77, respectively; or,
the light chain variable region comprising LCDR1, LCDR2 and LCDR3 consisting of the amino acid sequences set forth in SEQ ID NOs: 94, 116 and 138, respectively; and/or, the heavy chain variable region comprising HCDR1, HCDR2 and HCDR3 consisting of the amino acid sequences set forth in SEQ ID NOs: 23, 51 and 77, respectively; or,
the light chain variable region comprising LCDR1, LCDR2 and LCDR3 consisting of the amino acid sequences set forth in SEQ ID NOs: 94, 116 and 138, respectively; and/or, the heavy chain variable region comprising HCDR1, HCDR2 and HCDR3 consisting of the amino acid sequences set forth in SEQ ID NOs: 23, 50 and 77, respectively.

In a certain particular embodiment,
the light chain variable region comprises a VL consisting of the amino acid sequence set forth in SEQ ID NO: 171 or an amino acid sequence having at least 85%, 90%, 95%, 96%, 97%, 98% or 99% amino acid identity to SEQ ID NO: 171; the heavy chain variable region comprises a VH consisting of the amino acid sequence set forth in SEQ ID NO: 153 or an amino acid sequence having at least 85%, 90%, 95%, 96%, 97%, 98% or 99% amino acid identity to SEQ ID NO: 153; or,
the light chain variable region comprises a VL consisting of the amino acid sequence set forth in SEQ ID NO: 173 or an amino acid sequence having at least 85%, 90%, 95%, 96%, 97%, 98% or 99% amino acid identity to SEQ ID NO: 173; the heavy chain variable region comprises a VH consisting of the amino acid sequence set forth in SEQ ID NO: 155 or an amino acid sequence having at least 85%, 90%, 95%, 96%, 97%, 98% or 99% amino acid identity to SEQ ID NO: 155; or,
the light chain variable region comprises a VL consisting of the amino acid sequence set forth in SEQ ID NO: 175 or an amino acid sequence having at least 85%, 90%, 95%, 96%, 97%, 98% or 99% amino acid identity to SEQ ID NO: 175; the heavy chain variable region comprises a VH consisting of the amino acid sequence set forth in SEQ ID NO: 157 or an amino acid sequence having at least 85%, 90%, 95%, 96%, 97%, 98% or 99% amino acid identity to SEQ ID NO: 157; or,
the light chain variable region comprises a VL consisting of the amino acid sequence set forth in SEQ ID NO: 165 or an amino acid sequence having at least 85%, 90%, 95%, 96%, 97%, 98% or 99% amino acid identity to SEQ ID NO: 165; the heavy chain variable region comprises a VH consisting of the amino acid sequence set forth in SEQ ID NO: 148 or an amino acid sequence having at least 85%, 90%, 95%, 96%, 97%, 98% or 99% amino acid identity to SEQ ID NO: 148; or,
the light chain variable region comprises a VL consisting of the amino acid sequence set forth in SEQ ID NO: 174 or an amino acid sequence having at least 85%, 90%, 95%, 96%, 97%, 98% or 99% amino acid identity to SEQ ID NO: 174; the heavy chain variable region comprises a VH consisting of the amino acid sequence set forth in SEQ ID NO: 156 or an amino acid sequence having at least 85%, 90%, 95%, 96%, 97%, 98% or 99% amino acid identity to SEQ ID NO: 156; or,
the light chain variable region comprises a VL consisting of the amino acid sequence set forth in SEQ ID NO: 163 or an amino acid sequence having at least 85%, 90%, 95%, 96%, 97%, 98% or 99% amino acid identity to SEQ ID NO: 163; the heavy chain variable region comprises a VH consisting of the amino acid sequence set forth in SEQ ID NO: 146 or an amino acid sequence having at least 85%, 90%, 95%, 96%, 97%, 98% or 99% amino acid identity to SEQ ID NO: 146; or,
the light chain variable region comprises a VL consisting of the amino acid sequence set forth in SEQ ID NO: 166 or an amino acid sequence having at least 85%, 90%, 95%, 96%, 97%, 98% or 99% amino acid identity to SEQ ID NO: 166; the heavy chain variable region comprises a VH consisting of the amino acid sequence set forth in SEQ ID NO: 149 or an amino acid sequence having at least 85%, 90%, 95%, 96%, 97%, 98% or 99% amino acid identity to SEQ ID NO: 149; or,
the light chain variable region comprises a VL consisting of the amino acid sequence set forth in SEQ ID NO: 167 or an amino acid sequence having at least 85%, 90%, 95%, 96%, 97%, 98% or 99% amino acid identity to SEQ ID NO: 167; the heavy chain variable region comprises a VH consisting of the amino acid sequence set forth in SEQ ID NO: 150 or an amino acid sequence having at least 85%, 90%, 95%, 96%, 97%, 98% or 99% amino acid identity to SEQ ID NO: 150; or,
the light chain variable region comprises a VL consisting of the amino acid sequence set forth in SEQ ID NO: 168 or an amino acid sequence having at least 85%, 90%, 95%, 96%, 97%, 98% or 99% amino acid identity to SEQ ID NO: 168; the heavy chain variable region comprises a VH consisting of the amino acid sequence set forth in SEQ ID NO: 151 or an amino acid sequence having at least 85%, 90%, 95%, 96%, 97%, 98% or 99% amino acid identity to SEQ ID NO: 151; or,
the light chain variable region comprises a VL consisting of the amino acid sequence set forth in SEQ ID NO: 172 or an amino acid sequence having at least 85%, 90%, 95%, 96%, 97%, 98% or 99% amino acid identity to SEQ ID NO: 172; the heavy chain variable region comprises a VH consisting of the amino acid sequence set forth in SEQ ID NO: 154 or an amino acid sequence having at least 85%, 90%, 95%, 96%, 97%, 98% or 99% amino acid identity to SEQ ID NO: 154; or,
the light chain variable region comprises a VL consisting of the amino acid sequence set forth in SEQ ID NO: 177 or an amino acid sequence having at least 85%, 90%, 95%, 96%, 97%, 98% or 99% amino acid identity to SEQ ID NO: 177; the heavy chain variable region comprises a VH consisting of the amino acid sequence set forth in SEQ ID NO: 159 or an amino acid sequence having at least 85%, 90%, 95%, 96%, 97%, 98% or 99% amino acid identity to SEQ ID NO: 159; or,
the light chain variable region comprises a VL consisting of the amino acid sequence set forth in SEQ ID NO: 169 or an amino acid sequence having at least 85%, 90%, 95%, 96%, 97%, 98% or 99% amino acid identity to SEQ ID NO: 169; the heavy chain variable region comprises a VH consisting of the amino acid sequence set forth in SEQ ID NO: 152 or an amino acid sequence having at least 85%, 90%, 95%, 96%, 97%, 98% or 99% amino acid identity to SEQ ID NO: 152; or,
the light chain variable region comprises a VL consisting of the amino acid sequence set forth in SEQ ID NO: 180 or an amino acid sequence having at least 85%, 90%, 95%, 96%, 97%, 98% or 99% amino acid identity to SEQ ID NO: 180; the heavy chain variable region comprises a VH consisting of the amino acid sequence set forth in SEQ ID NO: 152 or an amino acid sequence having at least 85%, 90%, 95%, 96%, 97%, 98% or 99% amino acid identity to SEQ ID NO: 152; or,
the light chain variable region comprises a VL consisting of the amino acid sequence set forth in SEQ ID NO: 170 or an amino acid sequence having at least 85%, 90%, 95%, 96%, 97%, 98% or 99% amino acid identity to SEQ ID NO: 170; the heavy chain variable region comprises a VH consisting of the amino acid sequence set forth in SEQ ID NO: 152 or an amino acid sequence having at least 85%, 90%, 95%, 96%, 97%, 98% or 99% amino acid identity to SEQ ID NO: 152; or,
the light chain variable region comprises a VL consisting of the amino acid sequence set forth in SEQ ID NO: 178 or an amino acid sequence having at least 85%, 90%, 95%, 96%, 97%, 98% or 99% amino acid identity to SEQ ID NO: 178; the heavy chain variable region comprises a VH consisting of the amino acid sequence set forth in SEQ ID NO: 160 or an amino acid sequence having at least 85%, 90%, 95%, 96%, 97%, 98% or 99% amino acid identity to SEQ ID NO: 160; or,
the light chain variable region comprises a VL consisting of the amino acid sequence set forth in SEQ ID NO: 179 or an amino acid sequence having at least 85%, 90%, 95%, 96%, 97%, 98% or 99% amino acid identity to SEQ ID NO: 179; the heavy chain variable region comprises a VH consisting of the amino acid sequence set forth in SEQ ID NO: 161 or an amino acid sequence having at least 85%, 90%, 95%, 96%, 97%, 98% or 99% amino acid identity to SEQ ID NO: 161; or,
the light chain variable region comprises a VL consisting of the amino acid sequence set forth in SEQ ID NO: 179 or an amino acid sequence having at least 85%, 90%, 95%, 96%, 97%, 98% or 99% amino acid identity to SEQ ID NO: 179; the heavy chain variable region comprises a VH consisting of the amino acid sequence set forth in SEQ ID NO: 160 or an amino acid sequence having at least 85%, 90%, 95%, 96%, 97%, 98% or 99% amino acid identity to SEQ ID NO: 160.

In a certain particular embodiment, the antibody or the variant thereof includes a chimeric antibody, a humanized antibody or a fully human antibody.

Preferably, the structure of the antibody or the variant thereof is Fab, Fab', F(ab')2, Fv, IgG, Fd, dAb, or a bispecific or multispecific antibody comprising the antibody or the variant thereof, or a monoclonal or polyclonal antibody prepared from the above-mentioned antibody; the Fv is preferably scFv; preferably, the antibody or the variant thereof having the structure IgG comprises a light chain and a heavy chain:
the light chain comprises the amino acid sequence set forth in SEQ ID NO: 207 or an amino acid sequence having at least 85%, 90%, 95%, 96%, 97%, 98% or 99% amino acid identity to SEQ ID NO: 207; the heavy chain comprises the amino acid sequence set forth in SEQ ID NO: 189 or an amino acid sequence having at least 85%, 90%, 95%, 96%, 97%, 98% or 99% amino acid identity to SEQ ID NO: 189; or,
the light chain comprises the amino acid sequence set forth in SEQ ID NO: 209 or an amino acid sequence having at least 85%, 90%, 95%, 96%, 97%, 98% or 99% amino acid identity to SEQ ID NO: 209; the heavy chain comprises the amino acid sequence set forth in SEQ ID NO: 191 or an amino acid sequence having at least 85%, 90%, 95%, 96%, 97%, 98% or 99% amino acid identity to SEQ ID NO: 191; or,
the light chain comprises the amino acid sequence set forth in SEQ ID NO: 211 or an amino acid sequence having at least 85%, 90%, 95%, 96%, 97%, 98% or 99% amino acid identity to SEQ ID NO: 211; the heavy chain comprises the amino acid sequence set forth in SEQ ID NO: 193 or an amino acid sequence having at least 85%, 90%, 95%, 96%, 97%, 98% or 99% amino acid identity to SEQ ID NO: 193; or,
the light chain comprises the amino acid sequence set forth in SEQ ID NO: 201 or an amino acid sequence having at least 85%, 90%, 95%, 96%, 97%, 98% or 99% amino acid identity to SEQ ID NO: 201; the heavy chain comprises the amino acid sequence set forth in SEQ ID NO: 184 or an amino acid sequence having at least 85%, 90%, 95%, 96%, 97%, 98% or 99% amino acid identity to SEQ ID NO: 184; or,
the light chain comprises the amino acid sequence set forth in SEQ ID NO: 210 or an amino acid sequence having at least 85%, 90%, 95%, 96%, 97%, 98% or 99% amino acid identity to SEQ ID NO: 210; the heavy chain comprises the amino acid sequence set forth in SEQ ID NO: 192 or an amino acid sequence having at least 85%, 90%, 95%, 96%, 97%, 98% or 99% amino acid identity to SEQ ID NO: 192; or,
the light chain comprises the amino acid sequence set forth in SEQ ID NO: 199 or an amino acid sequence having at least 85%, 90%, 95%, 96%, 97%, 98% or 99% amino acid identity to SEQ ID NO: 199; the heavy chain comprises the amino acid sequence set forth in SEQ ID NO: 182 or an amino acid sequence having at least 85%, 90%, 95%, 96%, 97%, 98% or 99% amino acid identity to SEQ ID NO: 182; or,
the light chain comprises the amino acid sequence set forth in SEQ ID NO: 202 or an amino acid sequence having at least 85%, 90%, 95%, 96%, 97%, 98% or 99% amino acid identity to SEQ ID NO: 202; the heavy chain comprises the amino acid sequence set forth in SEQ ID NO: 185 or an amino acid sequence having at least 85%, 90%, 95%, 96%, 97%, 98% or 99% amino acid identity to SEQ ID NO: 185; or,
the light chain comprises the amino acid sequence set forth in SEQ ID NO: 203 or an amino acid sequence having at least 85%, 90%, 95%, 96%, 97%, 98% or 99% amino acid identity to SEQ ID NO: 203; the heavy chain comprises the amino acid sequence set forth in SEQ ID NO: 186 or an amino acid sequence having at least 85%, 90%, 95%, 96%, 97%, 98% or 99% amino acid identity to SEQ ID NO: 186; or,
the light chain comprises the amino acid sequence set forth in SEQ ID NO: 204 or an amino acid sequence having at least 85%, 90%, 95%, 96%, 97%, 98% or 99% amino acid identity to SEQ ID NO: 204; the heavy chain comprises the amino acid sequence set forth in SEQ ID NO: 187 or an amino acid sequence having at least 85%, 90%, 95%, 96%, 97%, 98% or 99% amino acid identity to SEQ ID NO: 187; or,
the light chain comprises the amino acid sequence set forth in SEQ ID NO: 208 or an amino acid sequence having at least 85%, 90%, 95%, 96%, 97%, 98% or 99% amino acid identity to SEQ ID NO: 208; the heavy chain comprises the amino acid sequence set forth in SEQ ID NO: 190 or an amino acid sequence having at least 85%, 90%, 95%, 96%, 97%, 98% or 99% amino acid identity to SEQ ID NO: 190; or,
the light chain comprises the amino acid sequence set forth in SEQ ID NO: 213 or an amino acid sequence having at least 85%, 90%, 95%, 96%, 97%, 98% or 99% amino acid identity to SEQ ID NO: 213; the heavy chain comprises the amino acid sequence set forth in SEQ ID NO: 195 or an amino acid sequence having at least 85%, 90%, 95%, 96%, 97%, 98% or 99% amino acid identity to SEQ ID NO: 195; or,
the light chain comprises the amino acid sequence set forth in SEQ ID NO: 205 or an amino acid sequence having at least 85%, 90%, 95%, 96%, 97%, 98% or 99% amino acid identity to SEQ ID NO: 205; the heavy chain comprises the amino acid sequence set forth in SEQ ID NO: 188 or an amino acid sequence having at least 85%, 90%, 95%, 96%, 97%, 98% or 99% amino acid identity to SEQ ID NO: 188; or,
the light chain comprises the amino acid sequence set forth in SEQ ID NO: 216 or an amino acid sequence having at least 85%, 90%, 95%, 96%, 97%, 98% or 99% amino acid identity to SEQ ID NO: 216; the heavy chain comprises the amino acid sequence set forth in SEQ ID NO: 188 or an amino acid sequence having at least 85%, 90%, 95%, 96%, 97%, 98% or 99% amino acid identity to SEQ ID NO: 188; or,
the light chain comprises the amino acid sequence set forth in SEQ ID NO: 206 or an amino acid sequence having at least 85%, 90%, 95%, 96%, 97%, 98% or 99% amino acid identity to SEQ ID NO: 206; the heavy chain comprises the amino acid sequence set forth in SEQ ID NO: 188 or an amino acid sequence having at least 85%, 90%, 95%, 96%, 97%, 98% or 99% amino acid identity to SEQ ID NO: 188; or,
the light chain comprises the amino acid sequence set forth in SEQ ID NO: 214 or an amino acid sequence having at least 85%, 90%, 95%, 96%, 97%, 98% or 99% amino acid identity to SEQ ID NO: 214; the heavy chain comprises the amino acid sequence set forth in SEQ ID NO: 196 or an amino acid sequence having at least 85%, 90%, 95%, 96%, 97%, 98% or 99% amino acid identity to SEQ ID NO: 196; or,
the light chain comprises the amino acid sequence set forth in SEQ ID NO: 215 or an amino acid sequence having at least 85%, 90%, 95%, 96%, 97%, 98% or 99% amino acid identity to SEQ ID NO: 215; the heavy chain comprises the amino acid sequence set forth in SEQ ID NO: 197 or an amino acid sequence having at least 85%, 90%, 95%, 96%, 97%, 98% or 99% amino acid identity to SEQ ID NO: 197; or,
the light chain comprises the amino acid sequence set forth in SEQ ID NO: 215; the heavy chain comprises the amino acid sequence set forth in SEQ ID NO: 196.

In the present invention, the "Fab fragment" consists of one light chain and the CH1 and variable region of one heavy chain. The heavy chain of a Fab molecule cannot form a disulfide bond with another heavy chain molecule. The "Fc" region contains two heavy chain fragments comprising the CH2 and CH3 domains of an antibody. The two heavy chain fragments are held together by two or more disulfide bonds and by hydrophobic interactions of the CH3 domains. The "Fab' fragment" contains one light chain and a portion of a heavy chain that comprises the VH domain and the CH1 domain and the region between the CH1 and CH2 domains, whereby an interchain disulfide bond can be formed between the two heavy chains of two Fab' fragments to form a F(ab')2 molecule. The "F(ab')2 fragment" contains two light chains and two heavy chains comprising a portion of the constant region between the CH1 and CH2 domains, whereby an interchain disulfide bond is formed between the two heavy chains. Therefore, the F(ab')2 fragment consists of two Fab' fragments held together by a disulfide bond between the two heavy chains. The term "Fv" means an antibody fragment consisting of the VL and VH domains of a single arm of an antibody, but lacking the constant region.

In the present invention, the scFv (single chain antibody fragment, single chain antibody) can be a conventional single chain antibody in the art, which comprises a heavy chain variable region, a light chain variable region and a short peptide of 15-20 amino acids; wherein the VL and VH domains are paired by a linker which enables them to be produced as a single polypeptide chain, thereby forming a monovalent molecule [see, e.g., Bird et al., Science 242:423-426 (1988) and Huston et al., Proc. Natl. Acad. Sci. USA 85:5879-5883 (1988)]. Such scFv molecules can have a general structure of NH2-VL-linker-VH-COOH or NH2-VH-linker-VL-COOH. Suitable linkers in the prior art consist of a repetitive G4S amino acid sequence or a variant thereof. For example, linkers having an amino acid sequence (G4S)4 or (G4S)3 may be used, and variants thereof may also be used.

In the present invention, the Fd can be a conventional antibody fragment in the art, which comprises the part of Fab except VL and CL, containing about 225 amino acid residues, including VH, CH1 and part of the hinge chain.

In the present invention, the dAb can be conventional in the art, which consists of a VH or VL domain, is some minimal functional antibody fragments, and retains complete antigen-binding specificity. Its molecular weight is about one tenth of that of a normal antibody.

The term "multispecific antibody" is used in its broadest sense to encompass antibodies with multi-epitope specificity. These multispecific antibodies include, but are not limited to: antibodies comprising a heavy chain variable region (VH) and a light chain variable region (VL), wherein the VH-VL unit has multi-epitope specificity; antibodies having two or more VL and VH regions, wherein each VH-VL unit binds to a different target or a different epitope of the same target; antibodies having two or more single variable regions, wherein each single variable region binds to a different target or a different epitope of the same target; and full-length antibodies, antibody fragments, bispecific antibodies (diabodies), triabodies, and antibody fragments that are covalently or non-covalently linked together.

The antibody or the variant thereof of the present invention includes a monoclonal antibody. The monoclonal antibody (or mAb or Ab) described in the present invention refers to an antibody obtained from a single clonal cell strain. The cell strain is not limited to eukaryotic, prokaryotic or phage clonal cell strains.

The second aspect of the present invention provides a chimeric antigen receptor comprising the antibody or the variant thereof described in the first aspect of the present invention.

The third aspect of the present invention provides an isolated nucleic acid encoding the antibody or the variant thereof according to the first aspect of the present invention, or the chimeric antigen receptor according to the second aspect of the present invention.

The fourth aspect of the present invention provides a recombinant expression vector comprising the isolated nucleic acid according to the third aspect of the present invention; preferably, the recombinant expression vector is a plasmid, a cosmid, a phage or a viral vector, wherein the viral vector is preferably a retroviral vector, a lentiviral vector, an adenoviral vector or an adeno-associated viral vector.

The fifth aspect of the present invention provides a transformant, comprising the recombinant expression vector according to the fourth aspect of the present invention in a host cell; preferably, the host cell is a prokaryotic cell or a eukaryotic cell; more preferably, the host cell is selected from a yeast cell, a mammalian cell or other cells suitable for the preparation of antibodies or antigen-binding fragments thereof; wherein the mammalian cell is, for example, a 293 cell or a CHO cell.

The sixth aspect of the present invention provides an immune cell comprising the chimeric antigen receptor described in the second aspect of the present invention; preferably, the immune cell is a T cell or an NK cell; more preferably, the NK cell is a peripheral blood NK cell, an umbilical cord blood-derived NK cell, an NK cell differentiated from stem cells or an NK cell line such as an NK-92 cell line.

The seventh aspect of the present invention provides a method for preparing an antibody targeting CD25 or a variant thereof, which method comprises culturing the transformant according to the fifth aspect of the present invention, and obtaining the antibody targeting CD25 or the variant thereof from the culture.

The eighth aspect of the present invention provides an antibody-drug conjugate comprising a cytotoxic agent and the antibody targeting CD25 or the variant thereof according to the first aspect of the present invention.

The ninth aspect of the present invention provides a pharmaceutical composition comprising the antibody targeting CD25 or the variant thereof according to the first aspect of the present invention, the isolated nucleic acid of the third aspect of the present invention, the recombinant expression vector of the fourth aspect of the present invention, the transformant of the fifth aspect of the present invention, the immune cell of the sixth aspect of the present invention and/or the antibody-drug conjugate according to the eighth aspect of the present invention, and a pharmaceutically acceptable carrier;
preferably, the pharmaceutical composition further contains one or more of the groups consisting of a hormone preparation, a targeting small molecule preparation, a proteasome inhibitor, an imaging agent, a diagnostic agent, a chemotherapeutic agent, an oncolytic drug, a cytotoxic agent, a cytokine, an activator of a costimulatory molecule, an inhibitor of an inhibitory molecule, and a vaccine.

In some embodiments, the pharmaceutical composition or pharmaceutical preparation of the present invention comprises a suitable pharmaceutically acceptable carrier, for example, a pharmaceutical auxiliary material, such as a pharmaceutical carrier and a pharmaceutical excipient known in the art, including a buffer. As used in the present invention, the "pharmaceutically acceptable carrier" or "pharmaceutical carrier" includes any and all solvents, dispersion media, isotonic agents, absorption delaying agents, etc. that are physiologically compatible. Pharmaceutical carriers suitable for use in the present invention can be sterile liquids, such as water and oils, including those of petroleum, animal, vegetable or synthetic origin, such as peanut oil, soybean oil, mineral oil and sesame oil. Water is a preferred carrier when the pharmaceutical composition is administered intravenously. Saline solutions, aqueous dextrose and glycerol solutions can also be used as liquid carriers, particularly for injectable solutions. Suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, etc. For the use of excipients and application thereof, reference can also be made to "Handbook of Pharmaceutical Excipients", fifth edition, R.C.Rowe, P.J.Seskey and S.C.Owen, Pharmaceutical Press, London, Chicago. The composition, if desired, can also contain small amounts of wetting agents or emulsifying agents, or pH buffers. These compositions can be in the form of solutions, suspensions, emulsions, tablets, pills, capsules, powders, sustained-release formulations, etc. Oral formulation can contain a standard pharmaceutical carrier and/or excipient such as pharmaceutical grade mannitol, lactose, starch, magnesium stearate and saccharin. The pharmaceutical preparation or pharmaceutical composition comprising the antibody or the antigen-binding fragment thereof of the present invention can be prepared by mixing the antibody or the antigen-binding fragment thereof of the present invention having the desired purity with one or more optional pharmaceutical auxiliary materials (Remington's Pharmaceutical Sciences, 16th edition, Osol, A. ed. (1980)), preferably in the form of a lyophilized preparation or an aqueous solution. The pharmaceutical composition or preparation of the present invention may also contain more than one active ingredient, which is required for the particular indication being treated, preferably those having complementary activities that do not adversely affect each other. For example, it is desirable to also provide other anti-infective active ingredients, such as other antibodies, anti-infective active agents, small molecule drugs or immunomodulators. Such active ingredients are suitably present in combination in an amount effective for the intended use. Sustained-release preparations can be prepared. Suitable examples of sustained-release preparations include semipermeable matrices of solid hydrophobic polymers containing the antibody or the antigen-binding fragment thereof of the present invention, which matrices are in the form of shaped articles such as films or microcapsules.

Further provided is the use of the antibody targeting CD25 or the variant thereof according to any of the first aspect of the present invention, the chimeric antigen receptor according to the second aspect of the present invention, the isolated nucleic acid according to the third aspect of the present invention, the recombinant expression vector according to the fourth aspect of the present invention, the transformant according to the fifth aspect of the present invention, the immune cell according to the sixth aspect of the present invention, the antibody-drug conjugate according to the eighth aspect of the present invention and/or the pharmaceutical composition according to the ninth aspect of the present invention in the preparation of a drug for the diagnosis, prevention and/or treatment of a tumor.

The tenth aspect of the present invention provides a kit comprising the antibody targeting CD25 or the variant thereof according to the first aspect of the present invention, the chimeric antigen receptor according to the second aspect of the present invention, the isolated nucleic acid according to the third aspect of the present invention, the recombinant expression vector according to the fourth aspect of the present invention, the transformant according to the fifth aspect of the present invention, the immune cell according to the sixth aspect of the present invention, or the antibody-drug conjugate according to the eighth aspect of the present invention or the pharmaceutical composition according to the ninth aspect of the present invention;
preferably, the kit further comprises (i) a device for administering the antibody or the variant thereof, or the chimeric antigen receptor or the immune cell or the antibody-drug conjugate or the pharmaceutical composition; and/or (ii) instructions for use.

The eleventh aspect of the present invention provides a kit of parts comprising kit A and kit B, wherein
the kit A contains the antibody targeting CD25 or the antigen-binding fragment thereof according to the first aspect of the present invention, the chimeric antigen receptor according to the second aspect of the present invention, the isolated nucleic acid according to the third aspect of the present invention, the recombinant expression vector according to the fourth aspect of the present invention, the transformant according to the fifth aspect of the present invention, the immune cell according to the sixth aspect of the present invention, the antibody-drug conjugate according to the eighth aspect of the present invention and/or the pharmaceutical composition according to the ninth aspect of the present invention; and
the kit B contains other anti-tumor antibodies or a pharmaceutical composition comprising the other anti-tumor antibodies, and/or one or more of the groups consisting of a hormone preparation, a targeting small molecule preparation, a proteasome inhibitor, an imaging agent, a diagnostic agent, a chemotherapeutic agent, an oncolytic drug, a cytotoxic agent, a cytokine, an activator of a costimulatory molecule, an inhibitor of an inhibitory molecule, and a vaccine.

The twelfth aspect of the present invention provides a method for detecting CD25, which method comprises using the antibody targeting CD25 or the variant thereof according to the first aspect of the present invention, the chimeric antigen receptor according to the second aspect of the present invention, the immune cell according to the sixth aspect of the present invention, the antibody-drug conjugate according to the eighth aspect of the present invention and/or the pharmaceutical composition according to the ninth aspect of the present invention;
preferably, the detecting is for non-diagnostic and/or therapeutic purposes.

The thirteenth aspect of the present invention provides a method for preventing, treating or alleviating a disorder in a subject, which method comprises administering to the subject a therapeutically effective amount of the antibody targeting CD25 or the variant thereof according to any of the first aspect of the present invention, the isolated nucleic acid according to the third aspect of the present invention, the recombinant expression vector according to the fourth aspect of the present invention, the transformant according to the fifth aspect of the present invention, the immune cell according to the sixth aspect of the present invention, the antibody-drug conjugate according to the eighth aspect of the present invention and/or the pharmaceutical composition according to the ninth aspect of the present invention.

In some particular embodiments, the disorder in a subject is a proliferative disease, such as a tumor or a cancer. In some embodiments, the above-mentioned subject suffers from an established tumor, such as a solid tumor.

In some embodiments, there is provided a method for reducing the number of intra-tumor or tumor-infiltrating Treg cells in a subject; and in some embodiments, there is provided a method for eliminating or inhibiting the activity of intra-tumor or tumor-infiltrating Treg cells in a subject, both methods comprising administering to the subject an effective amount of the antibody targeting CD25 or the variant thereof according to any of the first aspect of the present invention, the chimeric antigen receptor according to the second aspect of the present invention, the isolated nucleic acid according to the third aspect of the present invention, the recombinant expression vector according to the fourth aspect of the present invention, the transformant according to the fifth aspect of the present invention, the immune cell according to the sixth aspect of the present invention, the antibody-drug conjugate according to the eighth aspect of the present invention and/or the pharmaceutical composition according to the ninth aspect of the present invention.

In some embodiments, there is provided a method for increasing the Teff/Treg ratio in a tumor in a subject, which method comprises administering to the subject the antibody targeting CD25 or the variant thereof according to any of the first aspect of the present invention, the chimeric antigen receptor according to the second aspect of the present invention, the isolated nucleic acid according to the third aspect of the present invention, the recombinant expression vector according to the fourth aspect of the present invention, the transformant according to the fifth aspect of the present invention, the immune cell according to the sixth aspect of the present invention, the antibody-drug conjugate according to the eighth aspect of the present invention and/or the pharmaceutical composition according to the ninth aspect of the present invention.

In some embodiments, there is provided a method for enhancing ADCC against tumor cells in a subject, which method comprises administering to the subject the antibody targeting CD25 or the variant thereof according to any of the first aspect of the present invention, the chimeric antigen receptor according to the second aspect of the present invention, the isolated nucleic acid according to the third aspect of the present invention, the recombinant expression vector according to the fourth aspect of the present invention, the transformant according to the fifth aspect of the present invention, the immune cell according to the sixth aspect of the present invention, the antibody-drug conjugate according to the eighth aspect of the present invention and/or the pharmaceutical composition according to the ninth aspect of the present invention.

In some particular embodiments, the ADCC effect against tumor cells in the subject is enhanced.

In some embodiments, there is provided the use of the antibody targeting CD25 or the variant thereof according to any of the first aspect of the present invention, the chimeric antigen receptor according to the second aspect of the present invention, the isolated nucleic acid according to the third aspect of the present invention, the recombinant expression vector according to the fourth aspect of the present invention, the transformant according to the fifth aspect of the present invention, the immune cell according to the sixth aspect of the present invention, the antibody-drug conjugate according to the eighth aspect of the present invention and/or the pharmaceutical composition according to the ninth aspect of the present invention for the preparation of a drug for the prevention, treatment or alleviation of a disorder in a subject, for the preparation of a drug for the reduction of the number of intra-tumor or tumor-infiltrating Treg cells in a subject, for the preparation of a drug for the elimination or inhibition of the activity of intra-tumor or tumor-infiltrating Treg cells in a subject, for the preparation of a drug for the increase of the Teff/Treg ratio in a tumor in a subject, and for the preparation of a drug for the enhancement of ADCC against tumor cells in a subject.

In some particular embodiments, the disorder in the above-mentioned subject is a proliferative disorder (e.g., cancer or tumor) or the above-mentioned subject suffers from a proliferative disorder (e.g., cancer or tumor). The tumor includes, but is not limited to, carcinoma, lymphoma, leukemia, blastoma, and sarcoma. More particular examples of such cancers include squamous cell carcinoma, myeloma, small cell lung cancer, non-small cell lung cancer, glioma, hepatocellular carcinoma (HCC), Hodgkin's lymphoma, non-Hodgkin's lymphoma, acute myeloid leukemia (AML), multiple myeloma, gastrointestinal (tract) cancer, kidney cancer, ovarian cancer, hepatic cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, cervical cancer, brain cancer, gastric cancer, bladder cancer, liver cancer, breast cancer, colon cancer and head and neck cancer.

In some particular embodiments, the cancer or tumor may be a solid tumor, including but not limited to, sarcoma (including a cancer arising from transformed cells of mesenchymal origin in tissues (such as a cancellous bone, cartilage, fat, muscle, blood vessel, hematopoietic cell or fibrous connective tissue)), carcinoma (including a tumor arising from epithelial cells), mesothelioma, neuroblastoma, and retinoblastoma. Cancers involving solid tumors include, but are not limited to, brain cancer, lung cancer, gastric cancer, duodenal cancer, esophageal cancer, breast cancer, colorectal cancer, kidney cancer, bladder cancer, renal cancer, pancreatic cancer, prostate cancer, ovarian cancer, melanoma, oral cancer, sarcoma, eye cancer, thyroid cancer, urethral cancer, vaginal cancer, neck cancer, and lymphoma.

In some particular embodiments, the cancer involves a CD25-expressing tumor, including, but not limited to, lymphoma, such as Hodgkin's lymphoma, and lymphocytic leukemia, such as chronic lymphocytic leukemia (CLL).

On the basis of meeting common knowledge in the art, the above-mentioned various preferred conditions can be combined in any form, such that various preferred examples of the present invention are obtained.

Reagents and raw materials used in the present invention are all commercially available. The present invention has the positive improvement effects as follows:
the monoclonal antibody targeting CD25 of the present invention is a naturally occurring antibody that has a binding activity against human CD25 and cynomolgus monkey CD25, and has a stronger ADCC effect after being modified with Fc; in addition, in-vitro experiments show that the antibody has a good effect of clearing Treg and lymphoma cells without blocking an IL-2 signaling pathway, does not affect the activity of Teff cells, and has good internalization activity, which is a potential antibody molecule suitable for the ADC drug development.

### Brief Description of the Drawings

FIG. 1 shows the binding of the anti-CD25 H2L2 antibodies to CHO-K1 cells overexpressing human CD25.
FIG. 2 shows the binding of the anti-CD25 H2L2 antibodies to HEK293 cells overexpressing monkey CD25.
FIG. 3 shows the binding of the anti-CD25 H2L2 antibodies to the tumor cell line SU-DHL-1 endogenously expressing human CD25.
FIG. 4 shows the binding of the anti-CD25 H2L2 antibodies to the tumor cell line Karpas299 endogenously expressing human CD25.
FIG. 5 shows the binding of the anti-CD25 H2L2 antibodies to human Treg cells and Teff cells.
FIG. 6 shows that the anti-CD25 H2L2 antibodies do not block the binding of human interleukin-2 to CHOK1-huCD25 cells.
FIG. 7 shows that the anti-CD25 H2L2 antibodies do not block the binding of human interleukin-2 to SU-DHL-1 cells.
FIG. 8 shows that the anti-CD25 H2L2 antibodies do not inhibit the phosphorylation of STATS, a protein downstream of the human interleukin-2 signaling pathway, as detected by AlphsLisa.
FIG. 9 shows that the anti-CD25 H2L2 antibodies do not inhibit the phosphorylation of STATS, a protein downstream of the human interleukin-2 signaling pathway, as detected by FACS.
FIG. 10 shows the ADCC effect of the anti-CD25 H2L2 antibodies against the SU-DHL-1 cell line, as detected using a reporter gene cell line.
FIG. 11 shows the ADCC effect of the anti-CD25 H2L2 antibodies against human Treg cells, as detected using a reporter gene cell line.
FIG. 12 shows the ADCC effect of the anti-CD25 H2L2 antibodies against human Teff cells, as detected using a reporter gene cell line.
FIG. 13 shows the endocytosis of Karpas 299 cells against the anti-CD25 H2L2 antibodies.
FIG. 14 shows the endocytosis of SU-DHL-1 cells against the anti-CD25 H2L2 antibodies.

### Detailed Description of Embodiments

The present invention is further described below by way of examples; however, the present invention is not limited to the scope of the described examples. For the experimental methods in which no specific conditions are specified in the following examples, selections are made according to conventional methods and conditions or according to the product instructions.

### Example 1. Obtaining of anti-CD25 antibody molecules

Experimental animals, which could be mice, rats, rabbits, sheep, camels, etc., were immunized with the CD25 recombinant protein to obtain antibody molecules that specifically bind to CD25. Usually, the resulting antibody molecules are of non-human origin. After obtaining non-human antibodies, these molecules need to be humanized using antibody engineering techniques to reduce immunogenicity and improve druggability. However, the process of humanizing antibodies has its technical complexity, and molecules that have been humanized tend to have reduced affinity for antigens. On the other hand, advances in transgenic technology have allowed the breeding of genetically engineered mice that carry the human immunoglobulin repertoire and in which their endogenous murine immunoglobulin repertoires are deleted. The Harbour H2L2 mouse (Harbour Antibodies BV) is a transgenic mouse carrying a human immunoglobulin repertoire. The antibody produced by this transgenic mouse has a fully human sequence, so no further humanization is required, greatly improving the efficiency of therapeutic antibody development.

### 1.1. Mouse immunization

The Harbour H2L2 mice were immunized in multiple rounds with a soluble recombinant human CD25-his fusion protein (Sino Biological, #10165-H08H) as an antigen. The antigenic protein was mixed with an immunoadjuvant to form an immunogenic reagent, which was then injected subcutaneously in the inguinal groin or injected intraperitoneally. In each round of immunization, each mouse received a total injection dose of 100 microliters. In the first round of immunization, each mouse was immunized with an immunogenic reagent prepared by mixing 50 micrograms of the antigenic protein with a complete Freund's adjuvant (Sigma, # F5881) at a volume ratio of 1 : 1. In each subsequent round of booster immunization, each mouse was immunized with an immunogenic reagent prepared by mixing 25 micrograms of the antigenic protein with Sigma Adjuvant System adjuvant (Sigma, #S6322). The interval between each round of booster immunization was at least two weeks, usually 6 to 7 rounds of booster immunizations. The immunization time was on day 0, 14, 28, 42, 56, 70, 84 and 98; and on day 49 and 77, the antibody titer in the serum of the mice was detected. 5 days before the isolation of splenic B cells from H2L2 mice, the final booster immunization was performed at a dose of 25 micrograms of the antigenic protein per mouse.

### 1.2. Detection of titer in serum

At specific time points, the mouse sera were collected, and the titer of the antibody binding to CD25 in the sera was detected by FACS.

cDNA encoding human CD25 (with Genbank accession number of NM000417) was obtained by gene synthesis and cloned into an expression vector pcDNA3.1, and then the expression vector was transfected into CHO cells to construct CHOK1-huCD25 cells highly expressing human CD25.

The serially diluted mouse sera were incubated with CHOK1-huCD25 cells at 4°C for 1 hour; after the cells were washed twice, a secondary antibody anti-rat IgG (H+L) (Life technologies, A11006) was added and incubated at 4°C for 1 hour, and after the cells were washed twice, the cells were resuspended and then detected by a flow cytometer (BD, CantoII). CHOK1 cells were used as background control.

### 1.3. Screening of anti-CD25 antibodies by hybridoma technique

A cDNA encoding cynomolgus monkey CD25 (with Genbank accession number of NM001283704) was obtained by gene synthesis and cloned into an expression vector pcDNA3.1, and then the expression vector was transfected into HEK293 cells to construct HEK293-cynoCD25 cells highly expressing cynomolgus monkey CD25.

The immunized mice with high titer in the sera were selected for one final immunization, and then the mice were sacrificed. The splenocytes and SP2/0 myeloma cells (ATCC, CRL-1581) were taken for electrofusion at a cell ratio of 4 : 1, wherein the electrofusion parameters were V1: 50 V, t1: 15 s, V2: 600 V, t2: 20 µs, t3: 0.5 s, n: 1, t4: 7 s, V+/-: +, fade: on. The cells were resuspended in a DMEM medium containing 20% FBS and HT and plated at 1 × 10⁵ cells/100 µL/well. After 24 hours, a DMEM medium containing 20% FBS and 2×HT was added at 100 µL/well for further culture. The supernatant was subsequently taken, and the titer of the antibody was detected. Generally, 9-15 days after the fusion, the supernatants from the mice immunized with the recombinant human CD25-his protein were initially screened using ELISA to detect the binding to the recombinant human CD25-his protein; and the positive clones were then further confirmed by FACS to detect the binding ability to the CHOK1 cell strain (CHOK1-huCD25) overexpressing human CD25 and the HEK293 cell strain (HEK293-cynoCD25) overexpressing cynomolgus monkey CD25. In addition, FACS was used to detect the blocking effects of the positive clones against the binding of interleukin-2 to CHOK1-huCD25. Positive wells without blocking effect were further subcloned by limiting dilution, and further screened by ELISA and FACS. The clones having good binding to human and monkey CD25 were selected for sequencing. In this example, the sequences of the variable domains of the anti-CD25 monoclonal antibody molecules obtained from the immunized Harbour H2L2 mice were human antibody sequences.

### 1.4. Preparation of fully human recombinant antibodies

After obtaining the light and heavy chain variable domain sequences encoding antibody molecules, the light and heavy chain variable domain sequences can be expressed in fusion with the corresponding human antibody light and heavy chain constant domain sequences using conventional recombinant DNA techniques to obtain recombinant antibody molecules. In this example, an antibody heavy chain variable domain (VH) sequence was obtained by gene synthesis and cloned into a mammalian cell expression plasmid vector encoding human IgG1 antibody heavy chain constant domain sequences to encode a full-length heavy chain that produces an IgG1 antibody. An antibody light chain variable domain (VL) sequence was obtained by gene synthesis and cloned into a mammalian cell expression plasmid vector encoding a human antibody Ig κ light chain constant domain sequence to encode a full-length light chain that produces an antibody. In this example, since the sequences of the variable domains of the anti-CD25 monoclonal antibody molecules obtained from the immunized Harbour H2L2 mice were human antibody sequences, a fully human anti-CD25 recombinant IgG1 antibody was also obtained in this example.

The plasmid encoding the antibody heavy chain and the plasmid encoding the antibody light chain were simultaneously transfected into mammalian host cells (such as human embryonic kidney cell HEK293), and a purified anti-CD25 recombinant antibody in which the light and heavy chains were correctly paired and assembled could be obtained using conventional recombinant protein expression and purification techniques. Specifically, HEK293 cells were subjected to scale-up culture in a FreeStyle^{™} F17 Expression Medium (Thermo ,#A1383504). Before the start of transient transfection, the cells were adjusted to a cell concentration of 6-8 × 10⁵ cells/ml, and cultured in a shaker at 37°C and 8% CO₂ for 24 hours at a cell concentration of 1.2 × 10⁶ cells/ml. 30 ml of the cultured cells was prepared. The above-mentioned plasmid encoding the antibody heavy chain and plasmid encoding the antibody light chain were mixed at a ratio of 2 : 3, a total of 30 µg of the plasmids was dissolved in 1.5 ml of Opti-MEM reduced serum medium (Thermo, 31985088), and the resulting mixture was filtered with a 0.22 µm filter membrane for sterilization. Another 1.5 ml of Opti-MEM was taken and dissolved in 120 µl of 1 mg/ml PEI (Polysciences, Inc #23966-2), and the resulting mixture was left to stand for 5 minutes. The PEI was slowly added to the plasmids, the resulting mixture was incubated at room temperature for 10 minutes, the plasmid and PEI mixed solution was slowly dripped into the culture flask while shaking the culture flask, and culture was performed in a shaker at 37°C and 8% CO₂ for 5 days. After 5 days, the cell viability was observed. The culture was collected, and centrifuged at a rotary speed of 3300 g for 10 minutes, and then the supernatant was taken; and then the supernatant was centrifuged at a high speed to remove impurities. A gravity column (Bio-Rad, #7311550) containing MabSelect^{™} (GE Healthcare Life Science, #71-5020-91 AE) was equilibrated with PBS (pH 7.4), that is, the column was rinsed with 2-5 times the column volume of PBS. The supernatant sample was passed through the column; and the column was rinsed with 5-10 times the column volume of PBS, the protein of interest was eluted with 0.1 M glycine with pH 3.5, then adjusted with Tris-HCl with pH 8.0 until neutrality, and finally transferred to a PBS buffer through concentration and liquid exchange by an ultrafiltration tube (Millipore, UFC901024) to obtain a purified anti-CD25 antibody solution. Finally, the concentration was determined using NanoDrop (Thermo Scientific^{™} NanoDrop^{™} One), and the purified anti-CD25 antibody solution was subpackaged and stored for later use.

### 1.5 Enhancement of ADCC effector function by mutations in Fc region

An antibody heavy chain variable domain (VH) sequence was obtained by gene synthesis and cloned into a mammalian cell expression plasmid vector encoding human IgG1 antibody heavy chain constant domain sequences, and mutations S239D and I332E (substitution of serine at position 239 with aspartic acid, and substitution of isoleucine at position 332 with glutamic acid, according to EU numbering) were introduced into the CH2 region of the IgG1 heavy chain constant regions to enhance the ADCC effector function. The plasmids encoding the antibody heavy chain and light chain were then transfected into mammalian host cells using the method described in Example 1.4 to prepare the recombinant antibody proteins.

### 1.6 Enhancement of ADCC effector function by defucosylation

In this example, the Fc conformation was changed by removing fucosyl on the antibody saccharide chain (N297) to enhance the ADCC effector function. Then, the plasmid encoding the IgG1 antibody heavy chain and the plasmid encoding the antibody light chain were simultaneously transfected into mammalian host cells using the method described in Example 1.4, into which 2-fluoro peracetylated fucose (2FF) (Sigma-Aldrich, #344827) was added, and then steps such as cell culture, collection and purification were performed according to the method described in Example 1.4. The addition of compound 2FF can inhibit the activity of glycosidase and inhibit the production of fucosyl on the saccharide chain.

The recombinant antibody produced in this example has the same amino acid sequence as the recombinant antibody produced in Example 1.4, but with different saccharide chain modifications. To distinguish different antibody protein samples having the same amino acid sequence produced by the two methods, this example uses "AF" as the suffix of the antibody sample name to indicate that the sample is a "low-fucosyl" or "defucosylated" sample.

For example, PR004639 is a recombinant antibody containing normal IgG1 constant regions, and a corresponding antibody protein sample having a normal saccharide chain structure was prepared using the method described in Example 1.4. PR004639AF is a defucosylated antibody protein sample that has the same amino acid sequence as PR004639, and that is obtained by using the method of this example.

In some examples, the amino acid sequence of the same antibody can be used to prepare an antibody sample (numbered PRxxxxxx, where xxxxxx is a number) by the method described in Example 1.4 , and can also be used to prepare an antibody sample (numbered PRxxxxxxAF, where xxxxxx is a number) by the method of this example, and these samples differ in saccharide chain structure.

### 1.7 Sequences of fully human anti-CD25 recombinant antibodies

The amino acid sequences of the light and heavy chain variable domains, the full-length amino acid sequences of the light chain, the full-length amino acid sequences of the heavy chain and the amino acid sequences of the CDRs defined according to rules of the Chothia definition for the anti-CD25 antibodies of the present invention are listed in Table 2-1.

The control antibody used in various examples of the present invention was RG6292, the sequence of which was from the patent application US 20190284287 A1, and the corresponding recombinant IgG1 antibody was numbered PR004639. The method described in Example 1.6 was used to prepare the defucosylated antibody protein sample PR004639AF, an analog of RG6292. The sequence of PR004639 (PR004639AF, RG6292) is set forth in Table 2-5.

The control antibody used in the examples of the present invention was daclizumab (trade name Zenapax, Roche); and the corresponding recombinant IgG1 antibody was numbered PR003689, with a heavy chain sequence set forth in SEQ ID NO.217 and a light chain sequence set forth in SEQ ID NO.218.

The control antibody used in the examples of the present invention was HuMax-Tac (Genmab), the sequence of which was from the patent application WO 2014057119 A1, and the corresponding recombinant IgG1 antibody was numbered PR007722.

### Example 2 Sequence analysis and sequence optimization of antibodies

The heavy chain variable domain sequence of the antibody is derived from events such as gene rearrangement of germline gene V, D and J gene fragments of the heavy chain gene group on chromosomes and somatic hypermutation; and the light chain variable domain sequence is derived from events such as gene rearrangement of germline gene V and J gene fragments of the light chain gene group and somatic hypermutation. Gene rearrangement and somatic hypermutation are main factors that increase antibody diversity. Antibodies derived from the same germline V gene fragment may also give rise to different sequences, but the overall similarity is high. The possible germline gene fragments in the event of gene rearrangement can be deduced from the variable domain sequence of the antibody using some algorithms, such as IMGT/DomainGapAlign (http://imgt.org/3Dstructure-DB/cgi/DomainGapAlign.cgi) or NCBI/IgBLAST (https://www.ncbi.nlm.nih.gov/igblast/). The antibody sequences in Example 1 and Table 2-1 were analyzed, with the germline V gene fragments of the heavy chain variable domain (VH) and light chain variable domain (VL) listed in Table 2-2.

After the translation synthesis of amino acid chains of proteins or polypeptides in cells, chemical modifications are sometimes introduced, called post-translational modifications (PTMs). For antibodies, the sites of some PTMs are very conserved. For example, the conserved amino acid asparagine Asn at position 297 (EU numbering) of the constant domain of human IgG1 antibodies is usually glycosylated to form a saccharide chain, and this saccharide chain structure is critical for the antibody structure and related effector functions. However, if a PTM is present in a variable domain of an antibody, particularly in an antigen-binding region (e.g., CDR), the presence of such a PTM can have a greater impact on the binding to an antigen, and can also cause a change in the physicochemical properties of the antibody. For example, glycosylation, deamidation, isomerization, oxidation, etc. may increase the instability or heterogeneity of antibody molecules, thereby increasing the difficulty and risk of antibody development. Thus avoiding some potential PTMs is very important for the development of therapeutic antibodies. With the accumulation of experience, it has been found that some PTMs are highly correlated with the composition of amino acid sequences, particularly the "pattern" of the composition of adjacent amino acids, which makes it possible to predict potential PTMs from the primary amino acid sequences of proteins. For example, an N-linked glycosylation site can be predicted according the sequence pattern N-x-S/T (asparagine in the first position, any amino acid other than proline in the second position, and serine or threonine in the third position). Amino acid sequence patterns that cause PTMs may be derived from germline gene sequences, for example, pattern NST where human germline gene fragment IGHV3-33 naturally has a glycosylation modification in the FR3 region; and they may also be derived from somatic hypermutation. The predicted PTMs in the variable domains VH and VL of the antibody of Example 1 are listed in Table 2-2. Specifically, NSS or NLT may be a glycosylation site, and NS or NT or NN may be a deamidation site.

The amino acid sequence patterns of PTMs can be disrupted by amino acid mutations, thereby reducing or removing the formation of particular PTMs. Depending on different antibody sequences and different PTM sequence patterns, there are different mutation design methods. One method is to replace the "hot spot" amino acid (such as N or S in the NS pattern) with an amino acid having similar physicochemical properties (e.g., mutation of N to Q), or to replace it with any other amino acid by saturation mutation. If the PTM sequence pattern is derived from somatic hypermutation and is not present in the germline gene sequences, another method may be to replace the sequence pattern with the corresponding germline gene sequence. In actual operation, multiple mutation design methods may be used for the same PTM sequence pattern.

New antibody molecules (called PTM variants) resulting from amino acid mutations in the sequences of the antibodies having potential PTM sites from Example 1 are listed in Table 2-3. The amino acid sequences of the light and heavy chain variable domains, the full-length amino acid sequences of the light chain, the full-length amino acid sequences of the heavy chain (human IgG1) and the amino acid sequences of CDRs defined according to rules of the Chothia definition for these PTM variants in this example are listed in Table 2-4. Purified recombinant antibodies were obtained from all designed PTM variants according to the method described in Example 1.4, and further verified in subsequent functional experiments.

**Table 2-1 Screened anti-CD25 hybridoma monoclonal antibodies and anti-CD25 recombinant antibodies**

| Clone No. | Antibody | Light chain | Heavy chain | Light chain variable region | Heavy chain variable region | Light chain CDR1 | Light chain CDR2 | Light chain CDR3 | Heavy chain CDR1 | Heavy chain CDR2 | Heavy chain CDR3 |
|---|---|---|---|---|---|---|---|---|---|---|---|
| 113D5A1 | PR006927 | 207 | 189 | 171 | 153 | 96 | 111 | 133 | 20 | 44 | 74 |
| 310D11F6 | PR005436 | 201 | 184 | 165 | 148 | 97 | 113 | 129 | 16 | 40 | 70 |
| 171B2D12 | PR006931 | 210 | 192 | 174 | 156 | 94 | 116 | 135 | 23 | 47 | 77 |
| 308B6F9 | PR005433 | 199 | 182 | 163 | 146 | 95 | 111 | 127 | 14 | 38 | 68 |
| 245E10F1 | PR005497 | 202 | 185 | 166 | 149 | 98 | 114 | 130 | 17 | 41 | 71 |
| 307B8G6 | PR006110 | 203 | 186 | 167 | 150 | 99 | 115 | 131 | 18 | 42 | 72 |
| 189F2G11 | PR006122 | 204 | 187 | 168 | 151 | 100 | 113 | 132 | 14 | 43 | 73 |
| 150G7E2 | PR006928 | 208 | 190 | 172 | 154 | 103 | 110 | 134 | 21 | 45 | 75 |
| 170H1E12 | PR006930 | 209 | 191 | 173 | 155 | 104 | 111 | 127 | 22 | 46 | 76 |
| 184B11A4 | PR006932 | 211 | 193 | 175 | 157 | 94 | 110 | 134 | 23 | 48 | 78 |
| 111C1D7 | PR007181 | 213 | 195 | 177 | 159 | 105 | 111 | 136 | 18 | 42 | 80 |
| 180F6E8 | PR006984 | 212 | 194 | 176 | 158 | 94 | 110 | 134 | 24 | 49 | 79 |
| 308B6F9 | PR005435 | 200 | 183 | 164 | 147 | 96 | 112 | 128 | 15 | 39 | 69 |

**Table 2-2 Germline gene analysis and post-translational modification (PTM) site analysis of anti-CD25 antibody sequences**

| Clone No. | Antibody | VH germline V gene | VL germline V gene | VH PTM | VL PTM |
|---|---|---|---|---|---|
| 310D11F6 | PR005436 | IGHV4-4 | IGKV2-28 | DG (HCDR1) | DG (LCDR1) |
| 171B2D12 | PR006931 | IGHV1-2 | IGKV1-5 | NS (HCDR2) | NS (LCDR3) |

**Table 2-3 Mutation site design of anti-CD25 antibody sequences**

| Initial antibody | PTM variant | Mutation site |
|---|---|---|
| PR005436 | PR006769 | VH:D26P; VL:G34V |
| | PR008197 | VH:D26P; VL:G34V,M94L |
| | PR006771 | VH:D26P; VL:D33R |
| PR006931 | PR008014 | VH:N54P, S60Y; VL:N92L |
| | PR008016 | VH:N54Q, S60Y; VL:N92M |
| | PR008015 | VH:N54P, S60Y; VL:N92M |

**Table 2-4 Antibodies resulting from mutation at PTM**

| Initial antibody | PTM variant | Light chain | Heavy chain | Light chain variable region | Heavy chain variable region | Light chain CDR1 | Light chain CDR2 | Light chain CDR3 | Heavy chain CDR1 | Heavy chain CDR2 | Heavy chain CDR3 |
|---|---|---|---|---|---|---|---|---|---|---|---|
| PR005436 | PR006769 | 205 | 188 | 169 | 152 | 101 | 113 | 129 | 19 | 40 | 70 |
| | PR008197 | 216 | 188 | 180 | 152 | 101 | 113 | 139 | 19 | 40 | 70 |
| | PR006771 | 206 | 188 | 170 | 152 | 102 | 113 | 129 | 19 | 40 | 70 |
| PR006931 | PR008014 | 214 | 196 | 178 | 160 | 94 | 116 | 137 | 23 | 50 | 77 |
| | PR008016 | 215 | 197 | 179 | 161 | 94 | 116 | 138 | 23 | 51 | 77 |
| | PR008015 | 215 | 196 | 179 | 160 | 94 | 116 | 138 | 23 | 50 | 77 |

**Table 2-5 Control Antibody**

| Alias | Antibody | Light chain | Heavy chain | Light chain variable region | Heavy chain variable region | Light chain CDR1 | Light chain CDR2 | Light chain CDR3 | Heavy chain CDR1 | Heavy chain CDR2 | Heavy chain CDR3 |
|---|---|---|---|---|---|---|---|---|---|---|---|
| RG6292 | PR004639 | 198 | 181 | 162 | 145 | 94 | 110 | 126 | 13 | 37 | 67 |

**Table 2-6 Specific sequences of antibodies**

| | | VH CDR1 | | VH CDR2 | | VH CDR3 |
|---|---|---|---|---|---|---|
| PR004639 | 13 | GGTFSSL | 37 | IPIFGT | 67 | GGSVSGTLVDFDI |
| PR005433 | 14 | GFTFSSY | 38 | SGRGGS | 68 | EGYSSSYYEYFQH |
| PR005435 | 15 | GDRVSNINA | 39 | YYRSKWY | 69 | DPWEAFDY |
| PR005436 | 16 | DGSISSSN | 40 | YHRGS | 70 | YYYDSSGYYYGY |
| PR005497 | 17 | GDSVSNNNA | 41 | HYRSDRSKW Y | 71 | DWDHDAFDI |
| PR006110 | 18 | GGSISSSN | 42 | YHSGS | 72 | DRNYGSGNYPHY YHYYGMDV |
| PR006122 | 14 | GFTFSSY | 43 | WYDGSN | 73 | AGKGELPFDY |
| PR006769 | 19 | PGSISSSN | 40 | YHRGS | 70 | YYYDSSGYYYGY |
| PR006771 | 19 | PGSISSSN | 40 | YHRGS | 70 | YYYDSSGYYYGY |
| PR006927 | 20 | GGSIRSY | 44 | YYSGS | 74 | DILTGYSDWFFDL |
| PR006928 | 21 | GFTFSNY | 45 | WYDGIN | 75 | EGAGDAFDI |
| PR006930 | 22 | EFTFSSY | 46 | SGSGGN | 76 | EAYSSSWYEYFQ H |
| PR006931 | 23 | GYTFTGY | 47 | NPNSGG | 77 | EGEGDAFDI |
| PR006932 | 23 | GYTFTGY | 48 | NSNSGD | 78 | EGAGDAFDF |
| PR006984 | 24 | GFTFSKY | 49 | WFDGIN | 79 | EGVGDAFDI |
| PR007181 | 18 | GGSISSSN | 42 | YHSGS | 80 | DRLVAVAGIFDY |
| PR008014 | 23 | GYTFTGY | 50 | NPPSGG | 77 | EGEGDAFDI |
| PR008015 | 23 | GYTFTGY | 50 | NPPSGG | 77 | EGEGDAFDI |
| PR008016 | 23 | GYTFTGY | 51 | NPQSGG | 77 | EGEGDAFDI |
| PR008197 | 19 | PGSISSSN | 40 | YHRGS | 70 | YYYDSSGYYYGY |
| | | VL CDR1 | | VL CDR2 | | VL CDR3 |
| PR004639 | 94 | RASQSISSWLA | 110 | KASSLES | 126 | QQYNIYPIT |
| PR005433 | 95 | RASQSISSNLA | 111 | GASTRAT | 127 | QQYNYWPLT |
| PR005435 | 96 | RASQSVSSNLA | 112 | AASTRAT | 128 | QQYNDWSIT |
| PR005436 | 97 | RSSQSLLHRDG YNYVD | 113 | LGSNRAS | 129 | MQALQTPLT |
| PR005497 | 98 | RASQSISGWLA | 114 | KASSLET | 130 | LQYNGYST |
| PR006110 | 99 | RASQNISSYLN | 115 | AASSLQS | 131 | QQSYSIPYT |
| PR006122 | 100 | RSSQSLLHSNG YNYLD | 113 | LGSNRAS | 132 | MQVLQTPYT |
| PR006769 | 101 | RSSQSLLHRDV YNYVD | 113 | LGSNRAS | 129 | MQALQTPLT |
| PR006771 | 102 | RSSQSLLHRRG YNYVD | 113 | LGSNRAS | 129 | MQALQTPLT |
| PR006927 | 96 | RASQSVSSNLA | 111 | GASTRAT | 133 | QQYNNWIT |
| PR006928 | 103 | RASQSFDNWL A | 110 | KASSLES | 134 | QQYNSYST |
| PR006930 | 104 | RASQSVSDTLA | 111 | GASTRAT | 127 | QQYNYWPLT |
| PR006931 | 94 | RASQSISSWLA | 116 | EASSLKS | 135 | HQYNSYST |
| PR006932 | 94 | RASQSISSWLA | 110 | KASSLES | 134 | QQYNSYST |
| PR006984 | 94 | RASQSISSWLA | 110 | KASSLES | 134 | QQYNSYST |
| PR007181 | 105 | RASQTVSSDLA | 111 | GASTRAT | 136 | QQSNDWPYT |
| PR008014 | 94 | RASQSISSWLA | 116 | EASSLKS | 137 | HQYLSYST |
| PR008015 | 94 | RASQSISSWLA | 116 | EASSLKS | 138 | HQYMSYST |
| PR008016 | 94 | RASQSISSWLA | 116 | EASSLKS | 138 | HQYMSYST |
| PR008197 | 101 | RSSQSLLHRDV YNYVD | 113 | LGSNRAS | 139 | LQALQTPLT |

### Example 3. Preparation of pre-expanded human Treg cells and pre-activated human Teff cells

For the preparation of pre-expanded human Treg cells, Treg cells were isolated from fresh human PBMC (EasySep^{™} Human CD4+CD127lowCD25+Regulatory T Cell Isolation Kit, #18063), to these cells were added Treg expansion microbeads (Miltenyi Biotec Treg Expansion Kit, human #130-095-345) and IL-2 (peprotech, 200-02), and the cells were incubated in an incubator with 5% CO₂ at 37°C for 10 days to obtain the pre-expanded human Treg cells.

For the preparation of pre-activated Teff cells, T cells were isolated from fresh human PBMC (Miltenyi, #130096535), and added to a 6-well plate coated with OKT3 (Invitrogen, #16-0037-85, 2 µg/ml) at a density of 1 × 10⁶ cells/ml, and then an anti-CD28 antibody (Invitrogen, #16-0289-85, 1 µg/ml) was added. After the cells were cultured in a cell incubator for 72 hours, IL-2 (peprotech 200-02, 10 ng/ml) was added, and the cells were cultured for 72 hours to obtain the pre-activated Teff cells.

### Example 4. Detection of binding ability of anti-CD25 antibodies to CD25 by FACS

This example was intended to study the in-vitro binding activity of H2L2 monoclonal antibodies against human CD25 to human/cynomolgus monkey CD25. Antibody binding experiments at the cellular level were performed by using a CHOK1 cell strain overexpressing human CD25 (CHOK1-huCD25, Harbour BioMed), a HEK293 cell strain overexpressing cynomolgus monkey CD25 (HEK293-cynoCD25, Harbour BioMed) and cell lines SU-DHL-1 (*ATCC*^{®}CRL-2955) and Karpas299 (CoBioer Biosciences Co., Ltd., CBP60271) highly expressing human CD25, as well as the pre-expanded human Treg cells and the pre-activated human Teff cells. Briefly, the CHOK1-huCD25 cells and HEK293-cynoCD25 cells were digested, the SU-DHL-1 cells, Karpas299 cells, Treg cells and Teff cells were collected, and the cells were resuspended in PBS containing 2% BSA. The cells were adjusted to a cell density of 1 × 10⁶ cells/mL, respectively. The cells were seeded in a 96-well V-bottom plate (Corning, #3894) at 100 µL cells/well, and then the antibody to be tested at 2×final concentration with 5-fold concentration gradient dilution and the positive control antibody RG6292 were added at 100 µL/well. The cells were incubated at 4°C for 1 hour in the dark. After the incubation, the cells were rinsed twice by adding pre-chilled PBS containing 2% BSA at 100 µL/well, and centrifuged at 500 g at 4°C for 5 minutes, and the supernatant was discarded. A fluorescent secondary antibody (Alexa Fluor 488-conjugated AffiniPure Goat Anti-Human IgG, Fcy Fragment Specific, Jackson, #109-545-098, 1 : 1000 dilution) was then added at 100 µL/well, and the cells were incubated at 4°C for 1 hour in the dark. The cells were washed twice by adding pre-chilled PBS containing 2% BSA at 100 µL/well, and centrifuged at 500 g at 4°C for 5 minutes, and the supernatant was discarded. Finally, the cells were resuspended in pre-chilled PBS containing 2% BSA at 200 µL/well, and the fluorescence signal values were read using a BD CantoII flow cytometer.

The binding of the antibodies to human CD25 and cynomolgus monkey CD25 on the cell surface and to CD25 on the surface of tumor cells SU-DHL-1 and Karpas is summarized as follows (Table 4-1, Table 4-2, Table 4-3, Table 4-4, Table 4-5, Table 4-6, Table 4-7, Table 4-8, and Table 4-9).

**Table 4-1 Binding of anti-CD25 antibodies to human CD25 and cynomolgus monkey CD25 on the cell surface and to CD25 expressed by tumor cells SU-DHL-1 and Karpas299**

| | CHOK1-huCD25 | | HEK293-cynoCD25 | | SU-DHL-1 | | Karpas299 | |
|---|---|---|---|---|---|---|---|---|
| Antibody | EC50 (nM) | Maximum MFI | EC50 (nM) | Maximum MFI | EC50 (nM) | Maximum MFI | EC50 (nM) | Maximum MFI |
| PR005433 | 8.19 | 22421 | 3.73 | 60515 | 4.90 | 44300 | 4.31 | 24893 |
| PR005435 | 15.25 | 31673 | 4.15 | 154283 | 97.58 | 19516 | 65.41 | 16150 |
| PR005436 | 0.86 | 49223 | 1.75 | 188922 | 1.40 | 33345 | 6718 | 32125 |
| RG6292 | 40.00 | 2665 | 40.00 | 2665 | 1.57 | 27996 | 14.29 | 20935 |

**Table 4-2 Binding of anti-CD25 antibodies to human CD25 and cynomolgus monkey CD25 on the cell surface and to CD25 expressed by tumor cells SU-DHL-1 and Karpas299**

| | CHOK1-huCD25 | | HEK293-cynoCD25 | | SU-DHL-1 | | Karpas299 | |
|---|---|---|---|---|---|---|---|---|
| Antibody | EC50 (nM) | Maximum MFI | EC50 (nM) | Maximum MFI | EC50 (nM) | Maximum MFI | EC50 (nM) | Maximum MFI |
| PR005497 | 2.73 | 60862 | 6.40 | 241654 | 2.40 | 42418 | 2.81 | 33087 |
| RG6292 | 45.78 | 4045 | 6.75 | 95048 | 1.35 | 21142 | 222 | 16596 |

**Table 4-3 Binding of anti-CD25 antibodies to cynomolgus monkey CD25 on the cell surface and to CD25 expressed by tumor cells SU-DHL-1 and Karpas299**

| | HEK293-cynoCD25 | | SU-DHL-1 | | Karpas299 | |
|---|---|---|---|---|---|---|
| Antibody | EC50 (nM) | Maximum MFI | EC50 (nM) | Maximum MFI | EC50 (nM) | Maximum MFI |
| PR006927 | 17.47 | 90092 | 4.68 | 27285 | 2034 | 10039 |
| PR006930 | 325 | 145679 | 2.20 | 31989 | 212 | 16355 |
| PR006931 | 625 | 151664 | 8.48 | 33717 | 40.00 | 9449 |
| PR006932 | 3.92 | 150908 | 3.51 | 34355 | 291 | 12021 |
| PR006122 | 1.63 | 82014 | 1.53 | 43865 | 1.21 | 18303 |
| RG6292 | 4.33 | 124778 | 3.87 | 32914 | 30.00 | 11773 |

**Table 4-4 Binding of anti-CD25 antibodies to CD25 expressed by tumor cells SU-DHL-1 and Karpas299**

| | SU-DHL-1 | | Karpas299 | |
|---|---|---|---|---|
| Antibody | EC50 (nM) | Maximum MFI | EC50 (nM) | Maximum MFI |
| PR007181 | 1.68 | 32548 | 5.81 | 10168 |
| PR006928 | 1.85 | 34672 | 20.00 | 10602 |
| PR006984 | 6.00 | 35251 | 55.00 | 8716 |
| RG6292 | 1.40 | 30669 | 5.00 | 10110 |

**Table 4-5 Binding of anti-CD25 antibodies to cynomolgus monkey CD25 on the cell surface and to CD25 expressed by tumor cells SU-DHL-1 and Karpas299**

| | HEK293-cynoCD25 | | SU-DHL-1 | | Karpas299 | |
|---|---|---|---|---|---|---|
| Antibody | EC50 (nM) | Maximum MFI | EC50 (nM) | Maximum MFI | EC50 (nM) | Maximum MFI |
| PR006769 | 3.53 | 132071 | 2.94 | 32166 | 39.26 | 16811 |
| PR006771 | 5.35 | 136359 | 4.56 | 29821 | 40.00 | 8973 |
| RG6292 | 2092 | 89732 | 3.87 | 32914 | 30.00 | 11773 |

**Table 4-6 Binding of anti-CD25 antibodies to cynomolgus monkey CD25 on the cell surface and to CD25 expressed by tumor cells SU-DHL-1 and Karpas299**

| | HEK293-cynoCD25 | | SU-DHL-1 | | Karpas299 | |
|---|---|---|---|---|---|---|
| Antibody | EC50 (nM) | Maximum MFI | EC50 (nM) | Maximum MFI | EC50 (nM) | Maximum MFI |
| PR008014 | 1.63 | 31472 | 1.36 | 32812 | 30.00 | 9513 |
| PR008016 | 2.25 | 27895 | 4.02 | 34468 | 40.00 | 8600 |
| RG6292 | 8077 | 8591 | 1.28 | 29729 | 5.00 | 9555 |

**Table 4-7 Binding of anti-CD25 antibodies to cynomolgus monkey CD25 on the cell surface and to CD25 expressed by tumor cells SU-DHL-1 and Karpas299**

| | HEK293-cynoCD25 | | SU-DHL-1 | | Karpas299 | |
|---|---|---|---|---|---|---|
| Antibody | EC50 (nM) | Maximum MFI | EC50 (nM) | Maximum MFI | EC50 (nM) | Maximum MFI |
| PR008197 | 225 | 25345 | 1.55 | 29019 | 8.00 | 9951 |
| RG6292 | 8077 | 8591 | 0.95 | 26261 | 3.41 | 11646 |

**Table 4-8 Binding of anti-CD25 antibody to CD25 expressed by tumor cells SU-DHL-1 and Karpas299**

| | SU-DHL-1 | | Karpas299 | |
|---|---|---|---|---|
| Antibody | EC50 (nM) | Maximum MFI | EC50 (nM) | Maximum MFI |
| PR008015 | 0.85 | 33925 | 20.00 | 10945 |
| RG6292 | 1.28 | 29729 | 5.00 | 9555 |

**Table 4-9 Binding of anti-CD25 antibody to CD25 expressed by tumor cells SU-DHL-1 and Karpas299**

| | SU-DHL-1 | | Karpas299 | |
|---|---|---|---|---|
| Antibody | EC50 (nM) | Maximum MFI | EC50 (nM) | Maximum MFI |
| PR006110 | 1.95 | 38936 | 1.73 | 14589 |
| RG6292 | 1.29 | 27519 | 11.82 | 9200 |

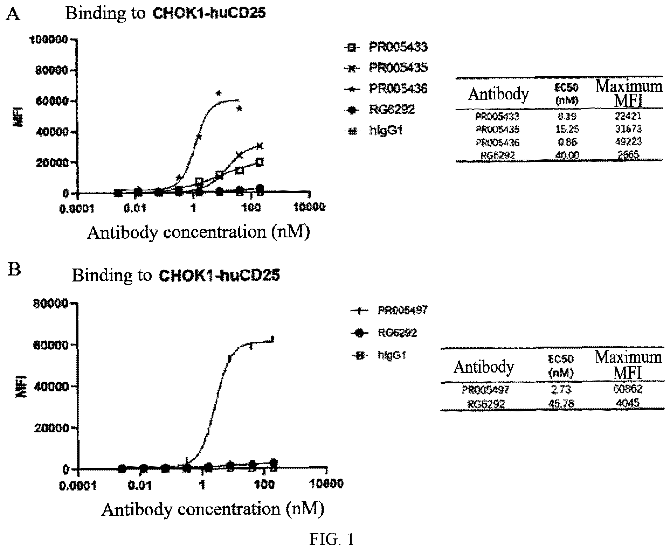

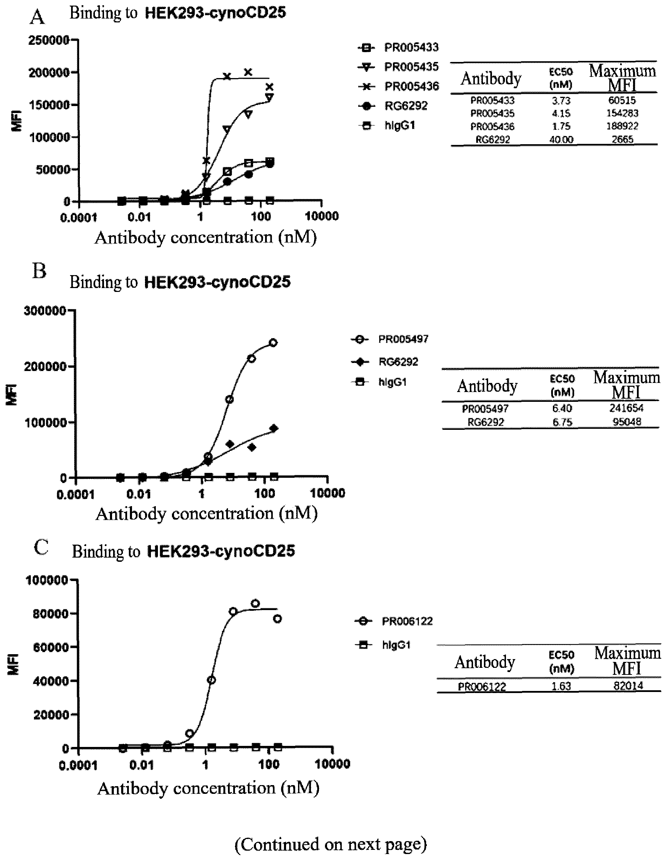

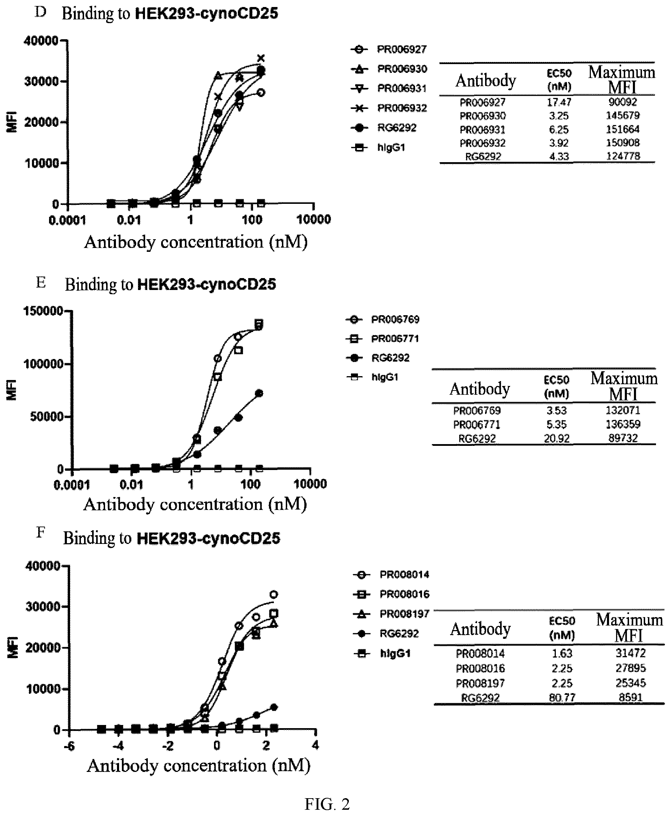

The binding of the antibodies to CHO-K1 cells overexpressing human CD25 is set forth in FIG. 1 (A in FIG. 1 and B in FIG. 1), Table 4-1 and Table 4-2, indicating that PR005433, PR005435, PR005436 and PR005497 have a higher binding activity compared with the control antibody. The binding of the antibodies to HEK293 cells overexpressing monkey CD25 is set forth in FIG. 2 (A in FIG. 2 to F in FIG. 2). and in conjunction with Table 4-1, Table 4-2 and Table 4-3, it can be seen that: the initial antibodies PR005433, PR005435, PR005436, PR005497, PR006122, PR006927, PR006930, PR006931 and PR006932 have good cross-binding activity against cynomolgus monkey CD25; in conjunction with Table 4-5 and Table 4-7, it can be seen that: the mutants PR006769, PR006771 and PR008197 resulting from PR005436 by mutations at PTMs have good cross-binding activity against cynomolgus monkey CD25; and in conjunction with Table 4-6, it can be seen that: the mutants PR008014 and PR008016 resulting from PR006931 by mutations at PTMs have good cross-binding activity against cynomolgus monkey CD25.

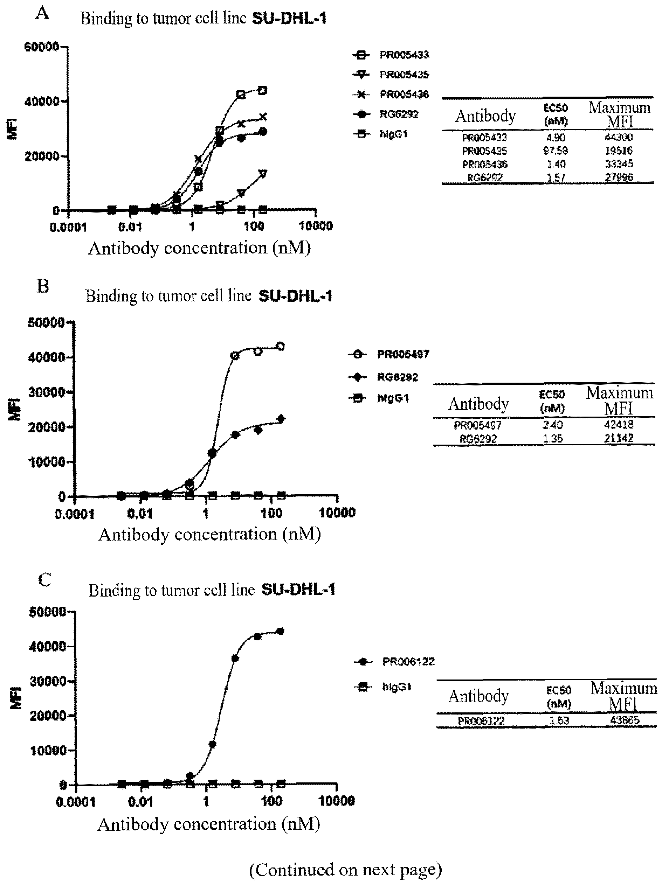

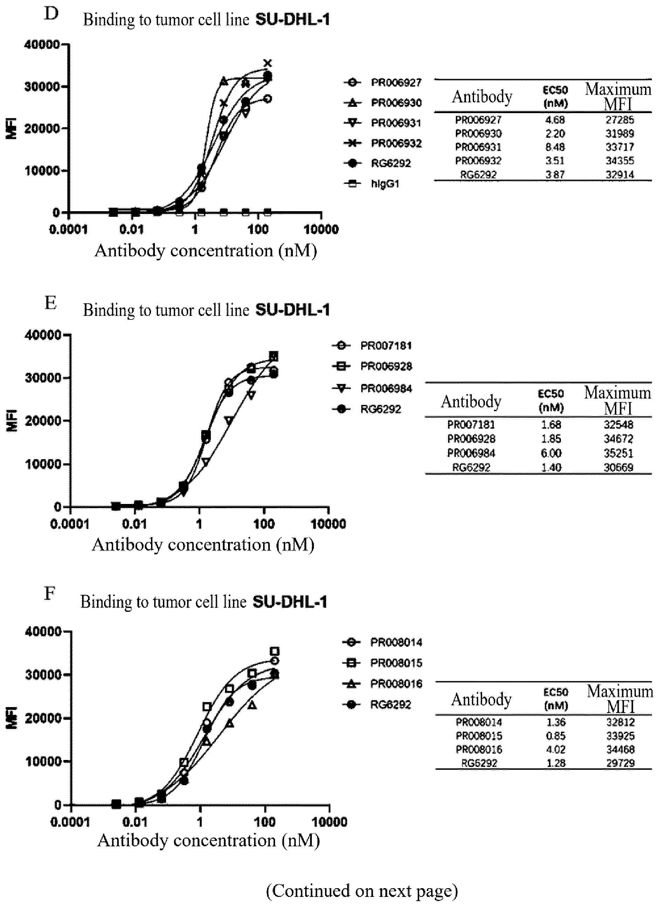

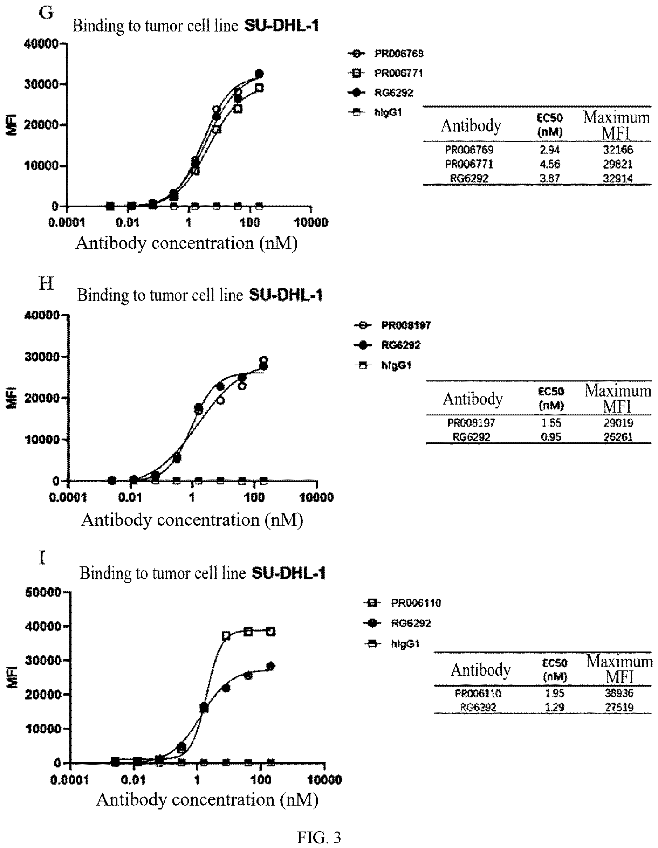

The binding of the antibodies to the tumor cell line SU-DHL-1 endogenously expressing human CD25 is set forth in FIG. 3 (A in FIG. 3 to I in FIG. 3), and in conjunction with Table 4-1, Table 4-2, Table 4-3, Table 4-4 and Table 4-9, it can be seen that: the initial antibodies PR005433, PR005436, PR005497, PR006110, PR006122, PR006927, PR006928, PR006930, PR006931, PR006932 and PR007181 have a high binding activity against SU-DHL-1; in conjunction with Table 4-1 and Table 4-4, it can be seen that: PR005435 and PR006984 have a weak binding activity; in conjunction with Table 4-5 and Table 4-7, it can be seen that: the mutants PR006769, PR006771 and PR008197 resulting from PR005436 by mutations at PTMs have a high binding activity against SU-DHL-1; and in conjunction with Table 4-6 and Table 4-8, it can be seen that: the mutants PR008014, PR008015 and PR008016 resulting from PR006931 by mutations at PTMs have a high binding activity against SU-DHL-1.

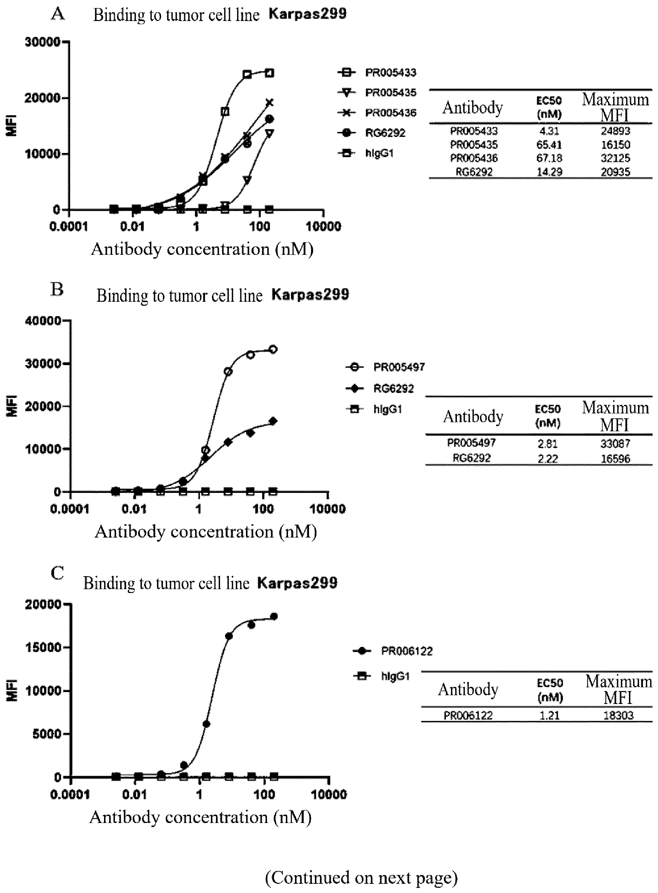

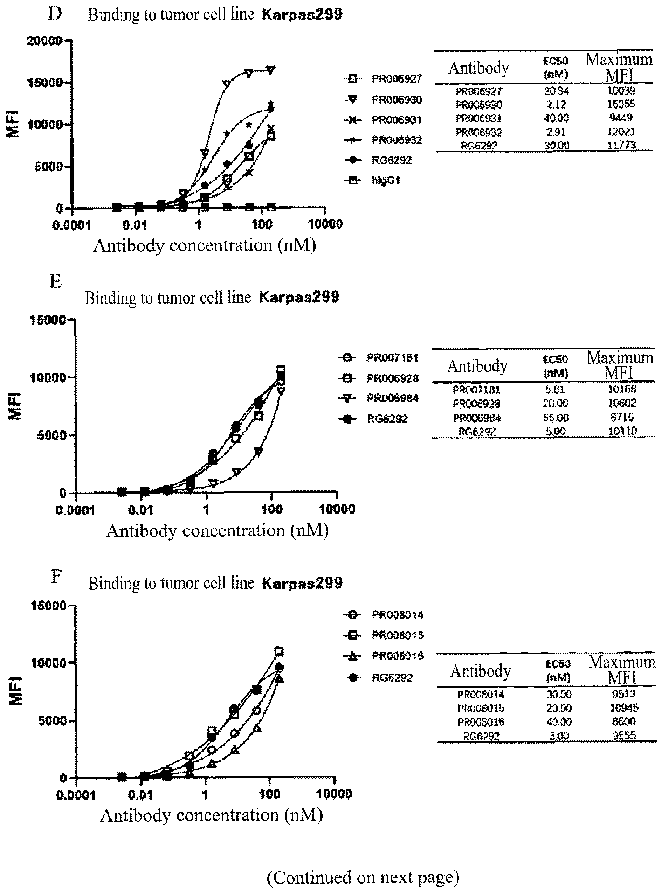

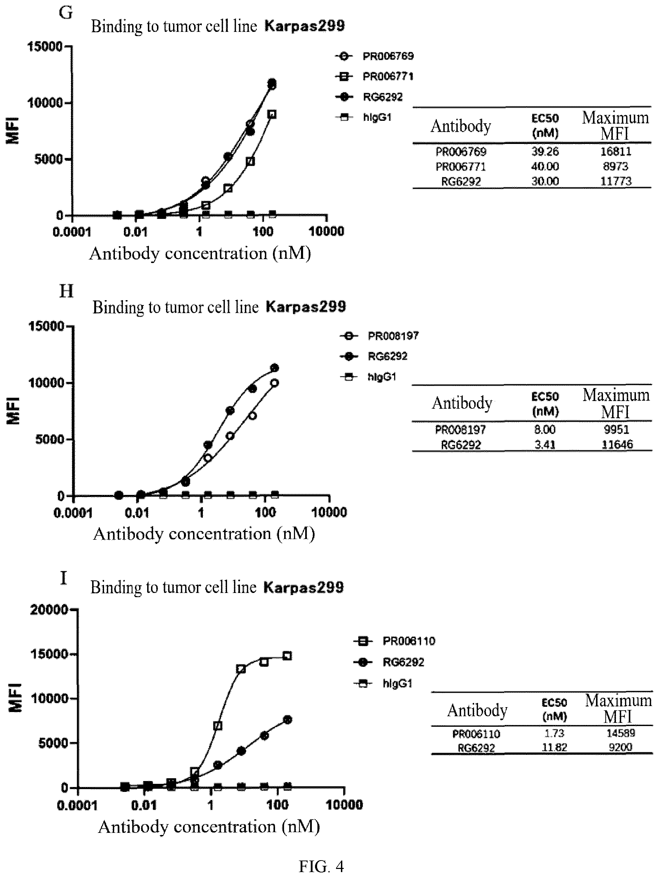

The binding of the antibodies to the tumor cell line Karpas299 endogenously expressing human CD25 is set forth in FIG. 4 (A in FIG. 4 to I in FIG. 4), and in conjunction with Table 4-1, Table 4-2, Table 4-3, Table 4-4 and Table 4-9, it can be seen that: the initial antibodies PR005433, PR005497, PR006110, PR006122, PR006927, PR006928, PR006930, PR006932 and PR007181 have a high binding activity against Karpas299; in conjunction with Table 4-1, Table 4-3 and Table 4-4, it can be seen that: PR005435, PR005436, PR006931 and PR006984 have a weak binding activity; in conjunction with Table 4-5, it can be seen that: the mutants PR006769 and PR006771 resulting from PR005436 by mutations at PTMs are comparable to the control antibody RG6292 in the binding activity against Karpas299; according to Table 4-7, it can be seen that: PR008197 has a relatively weak binding activity compared with the control antibody; in conjunction with Table 4-6 and Table 4-8, it can be seen that: the mutant PR008015 resulting from PR006931 by mutations at PTMs has a high binding activity against Karpas299; and PR008014 and PR008016 have a slightly weak binding activity.

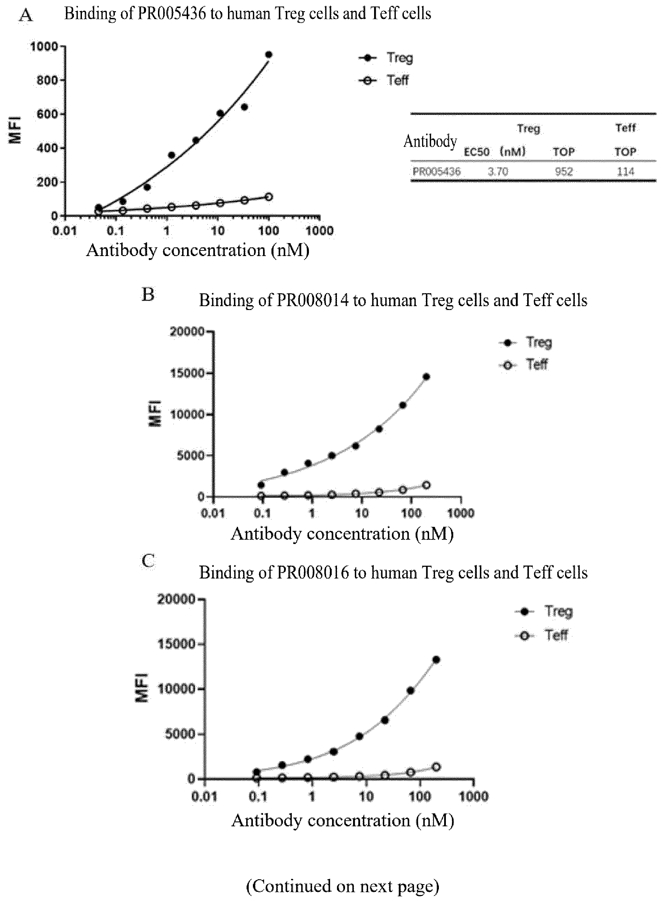

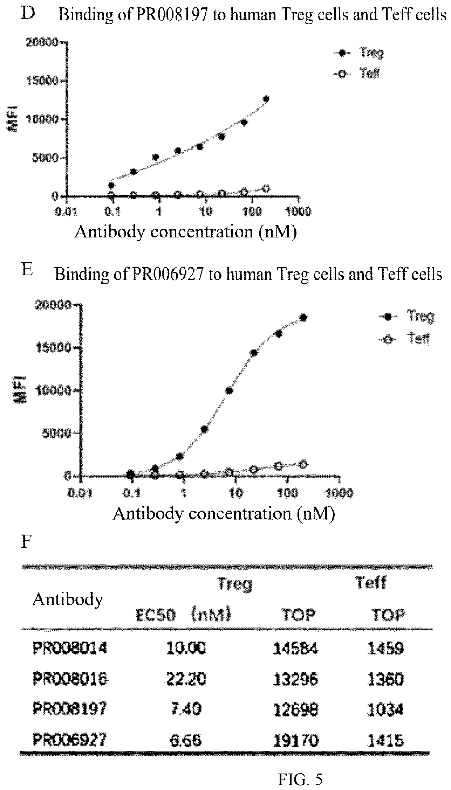

The binding of the antibodies to human Treg cells and Teff cells is set forth in FIG. 5 (A in FIG. 5 to F in FIG. 5), indicating that PR005436, PR008014, PR008016, PR008197 and PR006927 can bind to human Treg cells, and have a weak binding activity against human Teff cells.

### Example 5. Detection of blocking effects of anti-CD25 antibodies against the binding of interleukin-2 to CD25 by FACS

This example was intended to study the blocking effect of the anti-CD25 antibodies against the binding of interleukin-2 to CD25. The blocking effect of the antibody was detected using a CHOK1 cell strain overexpressing human CD25 (CHOK1-huCD25, Harbour BioMed) or a cell line SU-DHL-1 highly expressing human CD25 (*ATCC*^{®}CRL-2955) and biotinylated human interleukin-2 (Aero, II,2-H82F3) by FACS. Briefly, the CHOK1-huCD25 cells were digested or the SU-DHL-1 cells (*ATCC*^{®}CRL-2955) were collected, and the cells were resuspended in PBS containing 2% BSA. The cells were adjusted to a cell density of 1 × 10⁶ cells/mL, respectively. The cells were seeded in a 96-well V-bottom plate (Corning, #3894) at 90 µL cells/well, and then the antibody to be tested at 2×final concentration with 5-fold concentration gradient dilution and the control antibody RG6292 or PR003689 were added at 100 µL/well, then PBS containing biotinylated human interleukin-2 at a concentration of 10 µg/ml was added at 10 µL/well. The cells were incubated at 4°C for 1 hour in the dark. After the incubation, the cells were rinsed twice by adding pre-chilled PBS containing 2% BSA at 100 µL/well, and centrifuged at 500 g at 4°C for 5 minutes, and the supernatant was discarded. A fluorescent secondary antibody (eBioscience #12-4317-87, 1 : 200 dilution) was then added at 100 µL/well, and the cells were incubated at 4°C for 1 hour in the dark. The cells were washed twice by adding pre-chilled PBS containing 2% BSA at 100 µL/well, and centrifuged at 500 g at 4°C for 5 minutes, and the supernatant was discarded. Finally, the cells were resuspended in pre-chilled PBS containing 2% BSA at 200 µL/well, and the fluorescence signal values were read using a BD CantoII flow cytometer.

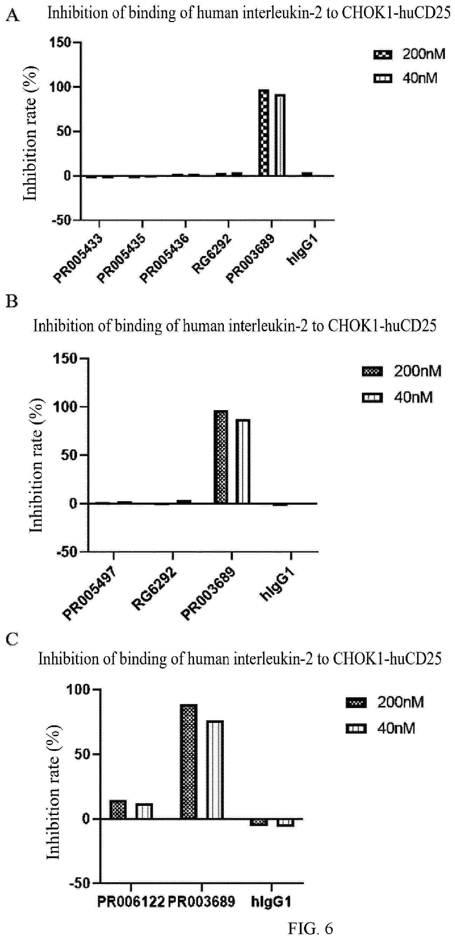

The blocking effects of the antibodies against the binding of human interleukin-2 to CHOK1-huCD25 cells as detected by FACS are set forth in FIG. 6 (A in FIG. 6 to C in FIG. 6), indicating that PR005433, PR005435, PR005436, PR005497 and PR006122 cannot block the binding of interleukin-2 to CD25 on the surface of CHOK1-huCD25 cells.

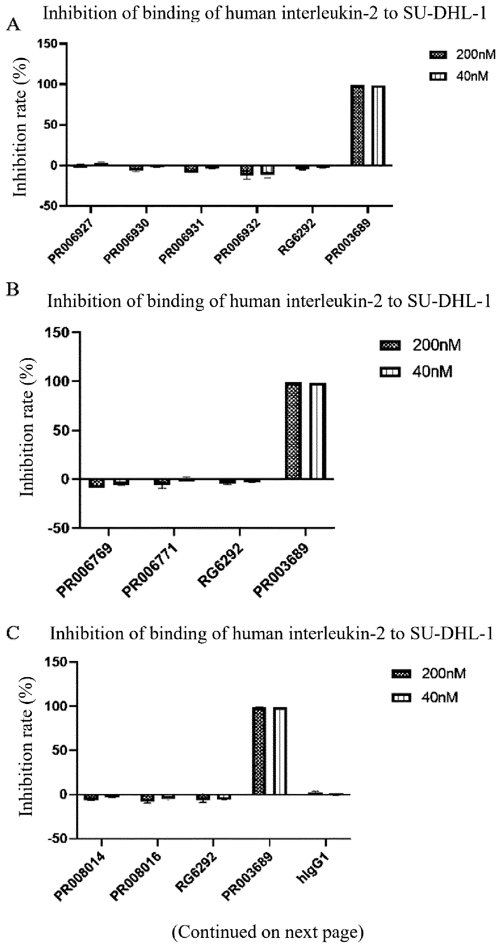

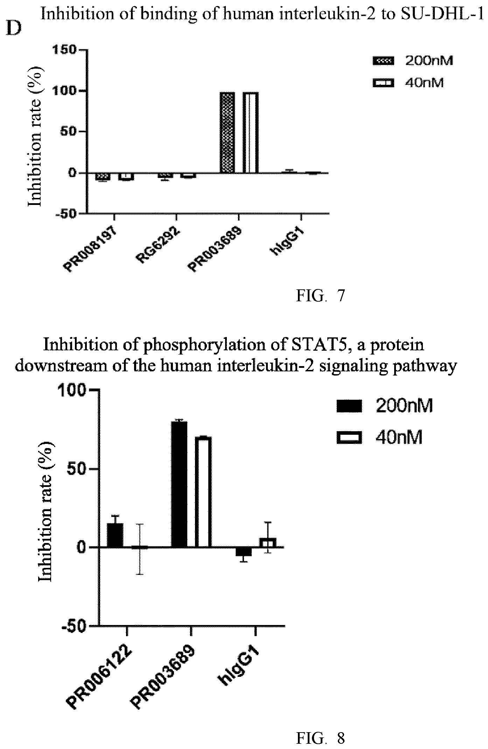

The blocking effects of the antibodies against the binding of human interleukin-2 to SU-DHL-1 (ATCC^{®}CRL-2955) cells as detected by FACS are set forth in FIG. 7 (A in FIG. 7 to D in FIG. 7), indicating that the initial antibodies PR006927, PR006930, PR006931 and PR006932 cannot block the binding of interleukin-2 to CD25 on the surface of SU-DHL-1 cells, mutants PR006769, PR006771 and PR008197 resulting from PR005436 by mutations at PTMs cannot block the binding of interleukin-2 to CD25 on the surface of SU-DHL-1 cells, and mutants PR008014 and PR008016 resulting from PR006931 by mutations at PTMs cannot block the binding of interleukin-2 to CD25 on the surface of SU-DHL-1 cells.

### Example 6. Detection of the effect of anti-CD25 antibodies on human interleukin-2 induced phosphorylation of STATS, downstream signaling molecule, in Teff cells by AlphaLisa (PerkinElimer, #ALSU-PST5-B500)

The pre-activated Teff cells were cultured with an IL-2-free medium for 4 hours, then collected, adjusted to a cell density of 2.5 × 10⁷ cells/ml, and seeded in a 384-well plate at 4 µL/well. The antibodies at 4×final concentration and the control antibody PR003689 were then added at 2 µL/well, and incubation was performed in an incubator for 1 hour. IL-2 at 5×final concentration was then added at 2 µL/well, and incubation was performed in the incubator for 10 minutes. 5×lysis solution was immediately added at 2 µL/well, and incubation was performed at room temperature for 15 minutes. Acceptor mixture was then added at 5 µL/well, incubation was performed at room temperature for 1 hour, Donor mixture was then added at 5 µL/well, and incubation was performed at room temperature for 1 hour. Signal values were read using PerkinElimer Envision.

The effect of the antibodies on the phosphorylation of STATS, a protein downstream of the human interleukin-2 signaling pathway as detected by AlphaLisa is set forth in FIG. 8, indicating that PR006122 has no inhibitory effect on the phosphorylation of STATS, a protein downstream of the human interleukin-2 signaling pathway in Teff cells.

### Example 7. Detection of the effect of anti-CD25 antibodies on human interleukin-2 induced phosphorylation of STATS, downstream signaling molecule, in Teff cells by FACS

The pre-activated Teff cells were cultured with an IL-2-free medium for 4 hours, then collected, and adjusted to a cell density of 2 × 10⁶ cells/mL. The cells were seeded in a 96-well V-bottom plate (Corning, #3894) at 50 µL cells/well, the antibody to be tested at 2×final concentration with 5-fold concentration gradient dilution and the control antibody PR003689 or RG6292 were then added at 50 µL/well, and incubation was performed in an incubator for 1 hour. 1 µg/ml IL-2 was then added at 1 µL/well, and incubation was performed in the incubator for 10 minutes. A fixation solution (Biolegend, #420801) was immediately added at 100 µL/well, incubation was performed at 37°C for 15 minutes, centrifugation was performed at 500 g at 4°C for 5 minutes, and the supernatant was discarded. The cells were rinsed by adding pre-chilled PBS containing 2% BSA at 100 µL/well, and centrifuged at 500 g at 4°C for 5 minutes, and the supernatant was discarded. A permeabilization buffer (Biolegend, #425401) was then added at 100 µL/well to resuspend the cells, and the cells were incubated in a refrigerator at -20°C overnight. The next day, the cells were taken out and centrifuged at 1000 g at 4°C for 5 minutes, the supernatant was discarded, the cells were rinsed twice by adding pre-chilled PBS containing 2% BSA at 100 µL/well, and centrifuged at 500 g at 4°C for 5 minutes, and the supernatant was discarded. An antibody against phosphorylated STATS (Biolegend, #936904) was added at 100 µL/well, and incubation was performed in a refrigerator at 4°C for 1 hour. The cells were rinsed twice by adding pre-chilled PBS containing 2% BSA at 100 µL/well, and centrifuged at 500 g at 4°C for 5 minutes, and the supernatant was discarded. Finally, the cells were resuspended in pre-chilled PBS containing 2% BSA at 200 µL/well, and the fluorescence signal values were read using a BD CantoII flow cytometer.

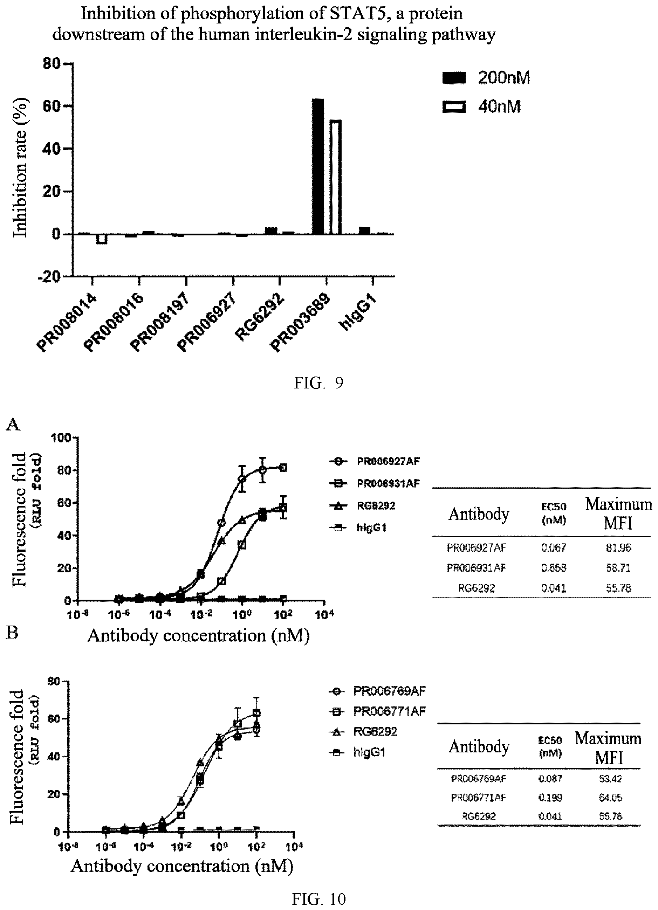

The results are set forth in FIG. 9, indicating that PR008014, PR008016, PR008197 and PR006927 have no inhibitory effect on the phosphorylation of STATS, a protein downstream of the human interleukin-2 signaling pathway in Teff cells.

### Example 8. Detection of ADCC effect of anti-CD25 antibodies against tumor cell line SU-DHL-1 using reporter gene cell line

SU-DHL-1 (ATCC^{®}CRL-2955) cells were collected and adjusted to a cell density of 1.2 × 10⁶ cells/mL, and seeded in a 96-well plate (PerkinElmer, 6005225) at 25 µL/well. A cDNA encoding human CD16a (with Genbank accession number of NM000569.8) and a cDNA encoding human NFAT (with Genbank accession number of NM_145912.8) were obtained by gene synthesis and cloned into an expression vector pcDNA3.1, and then the expression vector was transfected into Jurkat cells to construct a stable Jurkat/FCγRIII-NFAT cell strain.

Jurkat/FCγRIII-NFAT cells (Harbour BioMed) were collected and adjusted to a cell density of 1.2 × 10⁶ cells/mL, and seeded in the same 96-well plate at 25 µL/well. The antibodies at 2×final concentration and the control antibody RG6292 were then added at 50 µL/well, and the cells were incubated in an incubator with 5% CO₂ at 37°C for 6 hours. One-Glo (Promega, #E6120) was added at 60 µL/well, and the cells were placed at room temperature for 10 minutes in the dark. Signal values were read using PerkinElmer Envision.

The ADCC effects of the antibodies against SU-DHL-1 are summarized as follows (Table 8-1 and Table 8-2).

**Table 8-1 ADCC effect of anti-CD25 H2L2 antibodies against SU-DHL-1 cell line as detected using reporter gene cell line**

| | SU-DHL-1 | |
|---|---|---|
| Antibody | EC50 (nM) | Maximum MFI |
| PR006927AF | 0.067 | 81.96 |
| PR006931AF | 0.658 | 58.71 |
| RG6292 | 0.041 | 55.78 |

**Table 8-2 ADCC effect of anti-CD25 H2L2 antibodies against SU-DHL-1 cell line as detected using reporter gene cell line**

| | SU-DHL-1 | |
|---|---|---|
| Antibody | EC50 (nM) | Maximum MFI |
| PR006769AF | 0.087 | 53.42 |
| PR006771AF | 0.199 | 64.05 |
| RG6292 | 0.041 | 55.78 |

The results are set forth in FIG. 10 (A in FIG. 10 and B in FIG. 10), indicating that PR006927AF, PR006931AF, PR006769AF and PR006771AF have a strong ADCC effect against SU-DHL-1.

### Example 9. Detection of ADCC effect of anti-CD25 antibodies against pre-expanded human Treg cells and pre-activated human Teff cells using reporter gene cell line

Pre-expanded human Treg cells and pre-activated human Teff cells were collected, adjusted to a cell density of 1.2 × 10⁶ cells/mL, and seeded in a 96-well plate (PerkinElmer, 6005225) at 25 µL/well, respectively. Jurkat/FCγRIII-NFAT cells (Harbour BioMed) were collected and adjusted to a cell density of 1.2 × 10⁶ cells/mL, and seeded in the same 96-well plate at 25 µL/well. The antibody at 2×final concentration was then added at 50 µL/well, and the cells were incubated in an incubator with 5% CO₂ at 37°C for 6 hours. One-Glo (Promega, #E6120) was added at 60 µL/well, and the cells were placed at room temperature for 10 minutes in the dark. Signal values were read using PerkinElmer Envision.

The ADCC effects of the antibodies against Treg and Teff cells are summarized as follows (Table 9-1, Table 9-2 and Table 9-3).

**Table 9-1 ADCC effect of anti-CD25 H2L2 antibodies against Treg cells and Teff cells as detected using reporter gene cell line**

| | Treg | | Teff |
|---|---|---|---|
| Antibody | EC50 (nM) | Maximum MFI | Maximum MFI |
| PR005433 | 3.60 | 96.40 | 24.00 |
| PR005435 | 10.00 | 13.00 | 2.00 |
| PR005436 | 0.95 | 82.85 | 14.00 |

**Table 9-2 ADCC effect of anti-CD25 H2L2 antibodies against Treg cells and Teff cells as detected using reporter gene cell line**

| | Treg | | Teff |
|---|---|---|---|
| Antibody | EC50 (nM) | Maximum MFI | Maximum MFI |
| PR005497 | 0.05 | 88.90 | 30.19 |

**Table 9-3 ADCC effect of anti-CD25 H2L2 antibodies against Treg cells and Teff cells as detected using reporter gene cell line**

| | Treg | | Teff |
|---|---|---|---|
| Antibody | EC50 (nM) | Maximum MFI | Maximum MFI |
| PR006122 | 0.08 | 54.65 | 6.32 |

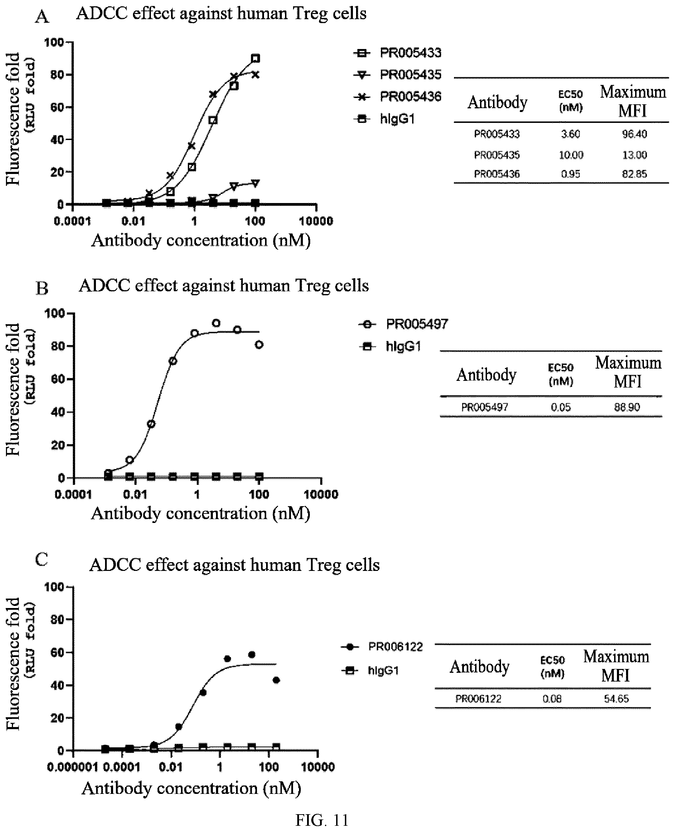

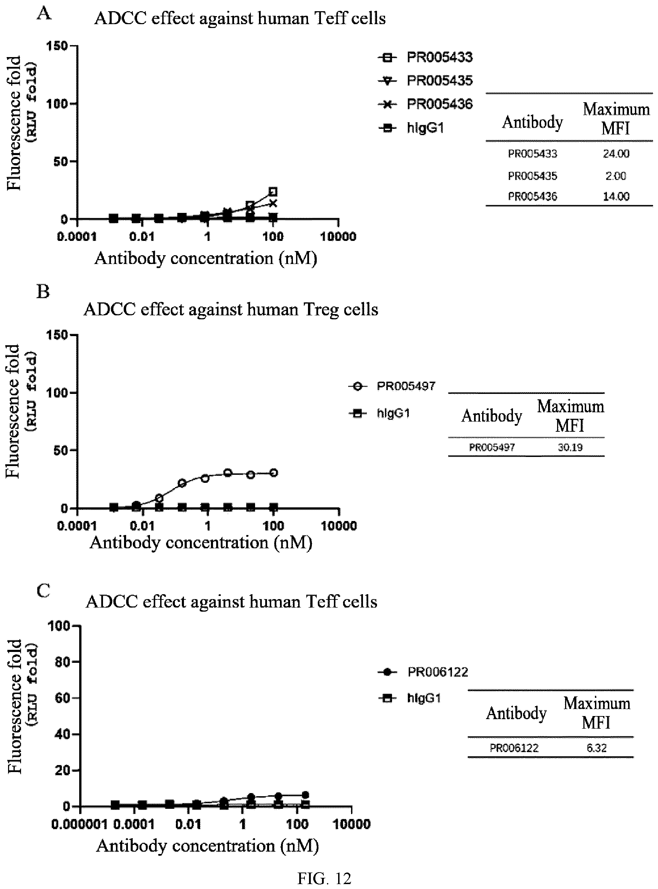

The results are set forth in FIG. 11 (A in FIG. 11 to C in FIG. 11) and FIG. 12 (A in FIG. 12 to C in FIG. 12), indicating that PR005433, PR005436, PR005497 and PR006122 have a strong ADCC effect against human Treg cells, while having no obvious ADCC effect against human Teff cells. PR005435 has a weak ADCC effect against human Treg cells.

### Example 10. Antibody internalization experiment

This example was intended to study antibody internalization. Tumor cell strains SU-DHL-1 (*ATCC*^{®}CRL-2955) and Karpas299 (CoBioer Biosciences Co., Ltd., CBP60271) highly expressing human CD25 were used to detect antibody internalization by FACS. The experimental method was as follows: SU-DHL-1 cells or Karpas299 cells were collected and resuspended in PBS containing 2% BSA. The cells were adjusted to a cell density of 1 × 10⁶ cells/mL, respectively. The cells were seeded in a 96-well V-bottom plate (Corning, #3894) at 100 µL cells/well, and then the antibody to be tested at 2×final concentration with 5-fold concentration gradient dilution and the control antibody PR007722 were added at 100 µL/well. The cells were incubated at 4°C for 1 hour in the dark. After the incubation, the cells were rinsed twice by adding pre-chilled PBS containing 2% BSA at 100 µL/well, and centrifuged at 500 g at 4°C for 5 minutes, and the supernatant was discarded. The cells were resuspended in 100 µL of PBS containing 2% BSA, and incubated in an incubator with 5% CO2 at 37°C for 4 hours. After the incubation, the cells were rinsed twice by adding pre-chilled PBS containing 2% BSA at 100 µL/well, and centrifuged at 500 g at 4°C for 5 minutes, and the supernatant was discarded. A fluorescent secondary antibody (Alexa Fluor 488-conjugated AffiniPure Goat Anti-Human IgG, Fcy Fragment Specific, Jackson, #109-545-098, 1 : 1000 dilution) was then added at 100 µL/well, and the cells were incubated at 4°C for 1 hour in the dark. The cells were washed twice by adding pre-chilled PBS containing 2% BSA at 100 µL/well, and centrifuged at 500 g at 4°C for 5 minutes, and the supernatant was discarded. Finally, the cells were resuspended in pre-chilled PBS containing 2% BSA at 200 µL/well, and the fluorescence signal values were read using a BD CantoII flow cytometer.

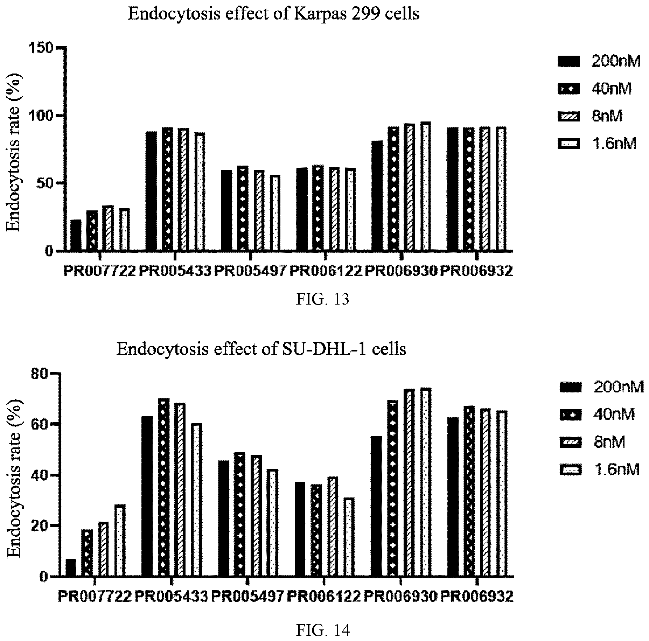

The results are set forth in FIG. 13 and FIG. 14, indicating that human tumor cell line Karpas299 has obvious endocytosis against PR005433, PR005497, PR006122, PR006930 and PR006932.

## Claims

1. An antibody targeting CD25 or a variant thereof, **characterized in that** the antibody comprises a light chain variable region and a heavy chain variable region, wherein the antibody is selected from the following groups:
the light chain variable region comprising LCDR1, LCDR2 and LCDR3 consisting of the amino acid sequences set forth in SEQ ID NOs: 96, 111 and 133, respectively; and/or, the heavy chain variable region comprising HCDR1, HCDR2 and HCDR3 consisting of the amino acid sequences set forth in SEQ ID NOs: 20, 44 and 74, respectively; or,
the light chain variable region comprising LCDR1, LCDR2 and LCDR3 consisting of the amino acid sequences set forth in SEQ ID NOs: 104, 111 and 127, respectively; and/or, the heavy chain variable region comprising HCDR1, HCDR2 and HCDR3 consisting of the amino acid sequences set forth in SEQ ID NOs: 22, 46 and 76, respectively; or,
the light chain variable region comprising LCDR1, LCDR2 and LCDR3 consisting of the amino acid sequences set forth in SEQ ID NOs: 94, 110 and 134, respectively; and/or, the heavy chain variable region comprising HCDR1, HCDR2 and HCDR3 consisting of the amino acid sequences set forth in SEQ ID NOs: 23, 48 and 78, respectively; or,
the light chain variable region comprising LCDR1, LCDR2 and LCDR3 consisting of the amino acid sequences set forth in SEQ ID NOs: 97, 113 and 129, respectively; and/or, the heavy chain variable region comprising HCDR1, HCDR2 and HCDR3 consisting of the amino acid sequences set forth in SEQ ID NOs: 16, 40 and 70, respectively; or,
the light chain variable region comprising LCDR1, LCDR2 and LCDR3 consisting of the amino acid sequences set forth in SEQ ID NOs: 94, 116 and 135, respectively; and/or, the heavy chain variable region comprising HCDR1, HCDR2 and HCDR3 consisting of the amino acid sequences set forth in SEQ ID NOs: 23, 47 and 77, respectively; or,
the light chain variable region comprising LCDR1, LCDR2 and LCDR3 consisting of the amino acid sequences set forth in SEQ ID NOs: 95, 111 and 127, respectively; and/or, the heavy chain variable region comprising HCDR1, HCDR2 and HCDR3 consisting of the amino acid sequences set forth in SEQ ID NOs: 14, 38 and 68, respectively; or,
the light chain variable region comprising LCDR1, LCDR2 and LCDR3 consisting of the amino acid sequences set forth in SEQ ID NOs: 98, 114 and 130, respectively; and/or, the heavy chain variable region comprising HCDR1, HCDR2 and HCDR3 consisting of the amino acid sequences set forth in SEQ ID NOs: 17, 41 and 71, respectively; or,
the light chain variable region comprising LCDR1, LCDR2 and LCDR3 consisting of the amino acid sequences set forth in SEQ ID NOs: 99, 115 and 131, respectively; and/or, the heavy chain variable region comprising HCDR1, HCDR2 and HCDR3 consisting of the amino acid sequences set forth in SEQ ID NOs: 18, 42 and 72, respectively; or,
the light chain variable region comprising LCDR1, LCDR2 and LCDR3 consisting of the amino acid sequences set forth in SEQ ID NOs: 100, 113 and 132, respectively; and/or, the heavy chain variable region comprising HCDR1, HCDR2 and HCDR3 consisting of the amino acid sequences set forth in SEQ ID NOs: 14, 43 and 73, respectively; or,
the light chain variable region comprising LCDR1, LCDR2 and LCDR3 consisting of the amino acid sequences set forth in SEQ ID NOs: 103, 110 and 134, respectively; and/or, the heavy chain variable region comprising HCDR1, HCDR2 and HCDR3 consisting of the amino acid sequences set forth in SEQ ID NOs: 21, 45 and 75, respectively; or,
the light chain variable region comprising LCDR1, LCDR2 and LCDR3 consisting of the amino acid sequences set forth in SEQ ID NOs: 105, 111 and 136, respectively; and/or, the heavy chain variable region comprising HCDR1, HCDR2 and HCDR3 consisting of the amino acid sequences set forth in SEQ ID NOs: 18, 42 and 80, respectively.

2. The antibody targeting CD25 or the variant thereof of claim 1, **characterized in that** the variant results from an addition, a deletion or a substitution of 1 to 3 amino acid residues in an amino acid sequence of the antibody targeting CD25; preferably, the variant results from a substitution of amino acid residues at position 1 of HCDR1 and at position 3 of HCDR2 in the heavy chain variable region of the antibody, and/or, a substitution of amino acid residues at positions 10-11 of LCDR1 and at position 1 or 4 of LCDR3 in the light chain variable region of the antibody; more preferably, the variant is selected from the following groups:
the light chain variable region comprising LCDR1, LCDR2 and LCDR3 consisting of the amino acid sequences set forth in SEQ ID NOs: 101, 113 and 129, respectively; and/or, the heavy chain variable region comprising HCDR1, HCDR2 and HCDR3 consisting of the amino acid sequences set forth in SEQ ID NOs: 19, 40 and 70, respectively; or,
the light chain variable region comprising LCDR1, LCDR2 and LCDR3 consisting of the amino acid sequences set forth in SEQ ID NOs: 101, 113 and 139, respectively; and/or, the heavy chain variable region comprising HCDR1, HCDR2 and HCDR3 consisting of the amino acid sequences set forth in SEQ ID NOs: 19, 40 and 70, respectively; or,
the light chain variable region comprising LCDR1, LCDR2 and LCDR3 consisting of the amino acid sequences set forth in SEQ ID NOs: 102, 113 and 129, respectively; and/or, the heavy chain variable region comprising HCDR1, HCDR2 and HCDR3 consisting of the amino acid sequences set forth in SEQ ID NOs: 19, 40 and 70, respectively; or,
the light chain variable region comprising LCDR1, LCDR2 and LCDR3 consisting of the amino acid sequences set forth in SEQ ID NOs: 94, 116 and 137, respectively; and/or, the heavy chain variable region comprising HCDR1, HCDR2 and HCDR3 consisting of the amino acid sequences set forth in SEQ ID NOs: 23, 50 and 77, respectively; or,
the light chain variable region comprising LCDR1, LCDR2 and LCDR3 consisting of the amino acid sequences set forth in SEQ ID NOs: 94, 116 and 138, respectively; and/or, the heavy chain variable region comprising HCDR1, HCDR2 and HCDR3 consisting of the amino acid sequences set forth in SEQ ID NOs: 23, 51 and 77, respectively; or,
the light chain variable region comprising LCDR1, LCDR2 and LCDR3 consisting of the amino acid sequences set forth in SEQ ID NOs: 94, 116 and 138, respectively; and/or, the heavy chain variable region comprising HCDR1, HCDR2 and HCDR3 consisting of the amino acid sequences set forth in SEQ ID NOs: 23, 50 and 77, respectively.

3. The antibody or the variant thereof of claim 1 or 2, **characterized in that**
the light chain variable region comprises a VL having an amino acid sequence set forth in SEQ ID NO: 171 or an amino acid sequence having at least 85%, 90%, 95%, 96%, 97%, 98% or 99% amino acid identity to the SEQ ID NO: 171; the heavy chain variable region comprises a VH having an amino acid sequence set forth in SEQ ID NO: 153 or an amino acid sequence having at least 85%, 90%, 95%, 96%, 97%, 98% or 99% amino acid identity to the SEQ ID NO: 153; or,
the light chain variable region comprises a VL having an amino acid sequence set forth in SEQ ID NO: 173 or an amino acid sequence having at least 85%, 90%, 95%, 96%, 97%, 98% or 99% amino acid identity to the SEQ ID NO: 173; the heavy chain variable region comprises a VH having an amino acid sequence set forth in SEQ ID NO: 155 or an amino acid sequence having at least 85%, 90%, 95%, 96%, 97%, 98% or 99% amino acid identity to the SEQ ID NO: 155; or,
the light chain variable region comprises a VL having an amino acid sequence set forth in SEQ ID NO: 175 or an amino acid sequence having at least 85%, 90%, 95%, 96%, 97%, 98% or 99% amino acid identity to the SEQ ID NO: 175; the heavy chain variable region comprises a VH having an amino acid sequence set forth in SEQ ID NO: 157 or an amino acid sequence having at least 85%, 90%, 95%, 96%, 97%, 98% or 99% amino acid identity to the SEQ ID NO: 157; or,
the light chain variable region comprises a VL having an amino acid sequence set forth in SEQ ID NO: 165 or an amino acid sequence having at least 85%, 90%, 95%, 96%, 97%, 98% or 99% amino acid identity to the SEQ ID NO: 165; the heavy chain variable region comprises a VH having an amino acid sequence set forth in SEQ ID NO: 148 or an amino acid sequence having at least 85%, 90%, 95%, 96%, 97%, 98% or 99% amino acid identity to the SEQ ID NO: 148; or,
the light chain variable region comprises a VL having an amino acid sequence set forth in SEQ ID NO: 174 or an amino acid sequence having at least 85%, 90%, 95%, 96%, 97%, 98% or 99% amino acid identity to the SEQ ID NO: 174; the heavy chain variable region comprises a VH having an amino acid sequence set forth in SEQ ID NO: 156 or an amino acid sequence having at least 85%, 90%, 95%, 96%, 97%, 98% or 99% amino acid identity to the SEQ ID NO: 156; or,
the light chain variable region comprises a VL having an amino acid sequence set forth in SEQ ID NO: 163 or an amino acid sequence having at least 85%, 90%, 95%, 96%, 97%, 98% or 99% amino acid identity to the SEQ ID NO: 163; the heavy chain variable region comprises a VH having an amino acid sequence set forth in SEQ ID NO: 146 or an amino acid sequence having at least 85%, 90%, 95%, 96%, 97%, 98% or 99% amino acid identity to the SEQ ID NO: 146; or,
the light chain variable region comprises a VL having an amino acid sequence set forth in SEQ ID NO: 166 or an amino acid sequence having at least 85%, 90%, 95%, 96%, 97%, 98% or 99% amino acid identity to the SEQ ID NO: 166; the heavy chain variable region comprises a VH having an amino acid sequence set forth in SEQ ID NO: 149 or an amino acid sequence having at least 85%, 90%, 95%, 96%, 97%, 98% or 99% amino acid identity to the SEQ ID NO: 149; or,
the light chain variable region comprises a VL having an amino acid sequence set forth in SEQ ID NO: 167 or an amino acid sequence having at least 85%, 90%, 95%, 96%, 97%, 98% or 99% amino acid identity to the SEQ ID NO: 167; the heavy chain variable region comprises a VH having an amino acid sequence set forth in SEQ ID NO: 150 or an amino acid sequence having at least 85%, 90%, 95%, 96%, 97%, 98% or 99% amino acid identity to the SEQ ID NO: 150; or,
the light chain variable region comprises a VL having an amino acid sequence set forth in SEQ ID NO: 168 or an amino acid sequence having at least 85%, 90%, 95%, 96%, 97%, 98% or 99% amino acid identity to the SEQ ID NO: 168; the heavy chain variable region comprises a VH having an amino acid sequence set forth in SEQ ID NO: 151 or an amino acid sequence having at least 85%, 90%, 95%, 96%, 97%, 98% or 99% amino acid identity to the SEQ ID NO: 151; or,
the light chain variable region comprises a VL having an amino acid sequence set forth in SEQ ID NO: 172 or an amino acid sequence having at least 85%, 90%, 95%, 96%, 97%, 98% or 99% amino acid identity to the SEQ ID NO: 172; the heavy chain variable region comprises a VH having an amino acid sequence set forth in SEQ ID NO: 154 or an amino acid sequence having at least 85%, 90%, 95%, 96%, 97%, 98% or 99% amino acid identity to the SEQ ID NO: 154; or,
the light chain variable region comprises a VL having an amino acid sequence set forth in SEQ ID NO: 177 or an amino acid sequence having at least 85%, 90%, 95%, 96%, 97%, 98% or 99% amino acid identity to the SEQ ID NO: 177; the heavy chain variable region comprises a VH having an amino acid sequence set forth in SEQ ID NO: 159 or an amino acid sequence having at least 85%, 90%, 95%, 96%, 97%, 98% or 99% amino acid identity to the SEQ ID NO: 159; or,
the light chain variable region comprises a VL having an amino acid sequence set forth in SEQ ID NO: 169 or an amino acid sequence having at least 85%, 90%, 95%, 96%, 97%, 98% or 99% amino acid identity to the SEQ ID NO: 169; the heavy chain variable region comprises a VH having an amino acid sequence set forth in SEQ ID NO: 152 or an amino acid sequence having at least 85%, 90%, 95%, 96%, 97%, 98% or 99% amino acid identity to the SEQ ID NO: 152; or,
the light chain variable region comprises a VL having an amino acid sequence set forth in SEQ ID NO: 180 or an amino acid sequence having at least 85%, 90%, 95%, 96%, 97%, 98% or 99% amino acid identity to the SEQ ID NO: 180; the heavy chain variable region comprises a VH having an amino acid sequence set forth in SEQ ID NO: 152 or an amino acid sequence having at least 85%, 90%, 95%, 96%, 97%, 98% or 99% amino acid identity to the SEQ ID NO: 152; or,
the light chain variable region comprises a VL having an amino acid sequence set forth in SEQ ID NO: 170 or an amino acid sequence having at least 85%, 90%, 95%, 96%, 97%, 98% or 99% amino acid identity to the SEQ ID NO: 170; the heavy chain variable region comprises a VH having an amino acid sequence set forth in SEQ ID NO: 152 or an amino acid sequence having at least 85%, 90%, 95%, 96%, 97%, 98% or 99% amino acid identity to the SEQ ID NO: 152; or,
the light chain variable region comprises a VL having an amino acid sequence set forth in SEQ ID NO: 178 or an amino acid sequence having at least 85%, 90%, 95%, 96%, 97%, 98% or 99% amino acid identity to the SEQ ID NO: 178; the heavy chain variable region comprises a VH having an amino acid sequence set forth in SEQ ID NO: 160 or an amino acid sequence having at least 85%, 90%, 95%, 96%, 97%, 98% or 99% amino acid identity to the SEQ ID NO: 160; or,
the light chain variable region comprises a VL having an amino acid sequence set forth in SEQ ID NO: 179 or an amino acid sequence having at least 85%, 90%, 95%, 96%, 97%, 98% or 99% amino acid identity to the SEQ ID NO: 179; the heavy chain variable region comprises a VH having an amino acid sequence set forth in SEQ ID NO: 161 or an amino acid sequence having at least 85%, 90%, 95%, 96%, 97%, 98% or 99% amino acid identity to the SEQ ID NO: 161; or,
the light chain variable region comprises a VL having an amino acid sequence set forth in SEQ ID NO: 179 or an amino acid sequence having at least 85%, 90%, 95%, 96%, 97%, 98% or 99% amino acid identity to the SEQ ID NO: 179; the heavy chain variable region comprises a VH having an amino acid sequence set forth in SEQ ID NO: 160 or an amino acid sequence having at least 85%, 90%, 95%, 96%, 97%, 98% or 99% amino acid identity to the SEQ ID NO: 160.

4. The antibody or the variant thereof of any one of claims 1-3, **characterized in that** the antibody is selected from the following groups: a chimeric antibody, a humanized antibody and a fully human antibody; preferably,
the structure of the antibody or the variant thereof is Fab, Fab', F(ab')2, Fv, IgG, Fd, dAb, or a bispecific or multispecific antibody comprising the antibody or the variant thereof, or a monoclonal or polyclonal antibody prepared from the above-mentioned antibody; the Fv is preferably scFv;
preferably, the antibody or the variant thereof having the structure IgG comprises a light chain and a heavy chain:
the light chain comprises an amino acid sequence set forth in SEQ ID NO: 207 or an amino acid sequence having at least 85%, 90%, 95%, 96%, 97%, 98% or 99% amino acid identity to the SEQ ID NO: 207; the heavy chain comprises an amino acid sequence set forth in SEQ ID NO: 189 or an amino acid sequence having at least 85%, 90%, 95%, 96%, 97%, 98% or 99% amino acid identity to the SEQ ID NO: 189; or,
the light chain comprises an amino acid sequence set forth in SEQ ID NO: 209 or an amino acid sequence having at least 85%, 90%, 95%, 96%, 97%, 98% or 99% amino acid identity to the SEQ ID NO: 209; the heavy chain comprises an amino acid sequence set forth in SEQ ID NO: 191 or an amino acid sequence having at least 85%, 90%, 95%, 96%, 97%, 98% or 99% amino acid identity to the SEQ ID NO: 191; or,
the light chain comprises an amino acid sequence set forth in SEQ ID NO: 211 or an amino acid sequence having at least 85%, 90%, 95%, 96%, 97%, 98% or 99% amino acid identity to the SEQ ID NO: 211; the heavy chain comprises an amino acid sequence set forth in SEQ ID NO: 193 or an amino acid sequence having at least 85%, 90%, 95%, 96%, 97%, 98% or 99% amino acid identity to the SEQ ID NO: 193; or,
the light chain comprises an amino acid sequence set forth in SEQ ID NO: 201 or an amino acid sequence having at least 85%, 90%, 95%, 96%, 97%, 98% or 99% amino acid identity to the SEQ ID NO: 201; the heavy chain comprises an amino acid sequence set forth in SEQ ID NO: 184 or an amino acid sequence having at least 85%, 90%, 95%, 96%, 97%, 98% or 99% amino acid identity to the SEQ ID NO: 184; or,
the light chain comprises an amino acid sequence set forth in SEQ ID NO: 210 or an amino acid sequence having at least 85%, 90%, 95%, 96%, 97%, 98% or 99% amino acid identity to the SEQ ID NO: 210; the heavy chain comprises an amino acid sequence set forth in SEQ ID NO: 192 or an amino acid sequence having at least 85%, 90%, 95%, 96%, 97%, 98% or 99% amino acid identity to the SEQ ID NO: 192; or,
the light chain comprises an amino acid sequence set forth in SEQ ID NO: 199 or an amino acid sequence having at least 85%, 90%, 95%, 96%, 97%, 98% or 99% amino acid identity to the SEQ ID NO: 199; the heavy chain comprises an amino acid sequence set forth in SEQ ID NO: 182 or an amino acid sequence having at least 85%, 90%, 95%, 96%, 97%, 98% or 99% amino acid identity to the SEQ ID NO: 182; or,
the light chain comprises an amino acid sequence set forth in SEQ ID NO: 202 or an amino acid sequence having at least 85%, 90%, 95%, 96%, 97%, 98% or 99% amino acid identity to the SEQ ID NO: 202; the heavy chain comprises an amino acid sequence set forth in SEQ ID NO: 185 or an amino acid sequence having at least 85%, 90%, 95%, 96%, 97%, 98% or 99% amino acid identity to the SEQ ID NO: 185; or,
the light chain comprises an amino acid sequence set forth in SEQ ID NO: 203 or an amino acid sequence having at least 85%, 90%, 95%, 96%, 97%, 98% or 99% amino acid identity to the SEQ ID NO: 203; the heavy chain comprises an amino acid sequence set forth in SEQ ID NO: 186 or an amino acid sequence having at least 85%, 90%, 95%, 96%, 97%, 98% or 99% amino acid identity to the SEQ ID NO: 186; or,
the light chain comprises an amino acid sequence set forth in SEQ ID NO: 204 or an amino acid sequence having at least 85%, 90%, 95%, 96%, 97%, 98% or 99% amino acid identity to the SEQ ID NO: 204; the heavy chain comprises an amino acid sequence set forth in SEQ ID NO: 187 or an amino acid sequence having at least 85%, 90%, 95%, 96%, 97%, 98% or 99% amino acid identity to the SEQ ID NO: 187; or,
the light chain comprises an amino acid sequence set forth in SEQ ID NO: 208 or an amino acid sequence having at least 85%, 90%, 95%, 96%, 97%, 98% or 99% amino acid identity to the SEQ ID NO: 208; the heavy chain comprises an amino acid sequence set forth in SEQ ID NO: 190 or an amino acid sequence having at least 85%, 90%, 95%, 96%, 97%, 98% or 99% amino acid identity to the SEQ ID NO: 190; or,
the light chain comprises an amino acid sequence set forth in SEQ ID NO: 213 or an amino acid sequence having at least 85%, 90%, 95%, 96%, 97%, 98% or 99% amino acid identity to the SEQ ID NO: 213; the heavy chain comprises an amino acid sequence set forth in SEQ ID NO: 195 or an amino acid sequence having at least 85%, 90%, 95%, 96%, 97%, 98% or 99% amino acid identity to the SEQ ID NO: 195; or,
the light chain comprises an amino acid sequence set forth in SEQ ID NO: 205 or an amino acid sequence having at least 85%, 90%, 95%, 96%, 97%, 98% or 99% amino acid identity to the SEQ ID NO: 205; the heavy chain comprises an amino acid sequence set forth in SEQ ID NO: 188 or an amino acid sequence having at least 85%, 90%, 95%, 96%, 97%, 98% or 99% amino acid identity to the SEQ ID NO: 188; or,
the light chain comprises an amino acid sequence set forth in SEQ ID NO: 216 or an amino acid sequence having at least 85%, 90%, 95%, 96%, 97%, 98% or 99% amino acid identity to the SEQ ID NO: 216; the heavy chain comprises an amino acid sequence set forth in SEQ ID NO: 188 or an amino acid sequence having at least 85%, 90%, 95%, 96%, 97%, 98% or 99% amino acid identity to the SEQ ID NO: 188; or,
the light chain comprises an amino acid sequence set forth in SEQ ID NO: 206 or an amino acid sequence having at least 85%, 90%, 95%, 96%, 97%, 98% or 99% amino acid identity to the SEQ ID NO: 206; the heavy chain comprises an amino acid sequence set forth in SEQ ID NO: 188 or an amino acid sequence having at least 85%, 90%, 95%, 96%, 97%, 98% or 99% amino acid identity to the SEQ ID NO: 188; or,
the light chain comprises an amino acid sequence set forth in SEQ ID NO: 214 or an amino acid sequence having at least 85%, 90%, 95%, 96%, 97%, 98% or 99% amino acid identity to the SEQ ID NO: 214; the heavy chain comprises an amino acid sequence set forth in SEQ ID NO: 196 or an amino acid sequence having at least 85%, 90%, 95%, 96%, 97%, 98% or 99% amino acid identity to the SEQ ID NO: 196; or,
the light chain comprises an amino acid sequence set forth in SEQ ID NO: 215 or an amino acid sequence having at least 85%, 90%, 95%, 96%, 97%, 98% or 99% amino acid identity to the SEQ ID NO: 215; the heavy chain comprises an amino acid sequence set forth in SEQ ID NO: 197 or an amino acid sequence having at least 85%, 90%, 95%, 96%, 97%, 98% or 99% amino acid identity to the SEQ ID NO: 197; or,
the light chain comprises an amino acid sequence set forth in SEQ ID NO: 215 or an amino acid sequence having at least 85%, 90%, 95%, 96%, 97%, 98% or 99% amino acid identity to the SEQ ID NO: 215; the heavy chain comprises an amino acid sequence set forth in SEQ ID NO: 196 or an amino acid sequence having at least 85%, 90%, 95%, 96%, 97%, 98% or 99% amino acid identity to the SEQ ID NO: 196.

5. A chimeric antigen receptor, **characterized by** comprising the antibody or the variant thereof of any one of claims 1-4.

6. An isolated nucleic acid, **characterized by** encoding the antibody or the variant thereof of any one of claims 1-4, or the chimeric antigen receptor of claim 5.

7. A recombinant expression vector, **characterized by** comprising the isolated nucleic acid of claim 6, wherein preferably, the recombinant expression vector is a plasmid, a cosmid, a phage or a viral vector, wherein the viral vector is preferably a retroviral vector, a lentiviral vector, an adenoviral vector or an adeno-associated viral vector.

8. A transformant, **characterized by** comprising the recombinant expression vector of claim 7 in a host cell, wherein preferably, the host cell is a prokaryotic cell or a eukaryotic cell; more preferably, the host cell is selected from a yeast cell, a mammalian cell or other cells suitable for the preparation of antibodies or antigen-binding fragments thereof; wherein the mammalian cell is, for example, a 293 cell or a CHO cell.

9. An immune cell, **characterized by** comprising the chimeric antigen receptor of claim 5, wherein preferably, the immune cell is a T cell or an NK cell; more preferably, the NK cell is a peripheral blood NK cell, an umbilical cord blood-derived NK cell, an NK cell differentiated from stem cells or an NK cell line such as an NK-92 cell line.

10. A method for preparing an antibody targeting CD25 or a variant thereof, **characterized in that** the method comprises culturing the transformant of claim 8, and obtaining the antibody targeting CD25 or the variant thereof from the culture.

11. An antibody-drug conjugate, **characterized by** comprising a cytotoxic agent and the antibody targeting CD25 or the variant thereof of any one of claims 1-4.

12. A pharmaceutical composition, **characterized by** comprising the antibody targeting CD25 or the variant thereof of any one of claims 1-4, the isolated nucleic acid of claim 6, the recombinant expression vector of claim 7, the transformant of claim 8, the immune cell of claim 9 and/or the antibody-drug conjugate of claim 11, and a pharmaceutically acceptable carrier;
wherein preferably, the pharmaceutical composition further contains one or more of the groups consisting of a hormone preparation, a targeting small molecule preparation, a proteasome inhibitor, an imaging agent, a diagnostic agent, a chemotherapeutic agent, an oncolytic drug, a cytotoxic agent, a cytokine, an activator of a costimulatory molecule, an inhibitor of an inhibitory molecule, and a vaccine.

13. Use of the antibody targeting CD25 or the variant thereof of any one of claims 1-4, the chimeric antigen receptor of claim 5, the isolated nucleic acid of claim 6, the recombinant expression vector of claim 7, the transformant of claim 8, the immune cell of claim 9, the antibody-drug conjugate of claim 11 and/or the pharmaceutical composition of claim 12 in the preparation of a drug for the diagnosis, prevention and/or treatment of a tumor.

14. A kit, **characterized by** comprising the antibody targeting CD25 or the variant thereof of any one of claims 1-4, the chimeric antigen receptor of claim 5, the isolated nucleic acid of claim 6, the recombinant expression vector of claim 7, the transformant of claim 8, the immune cell of claim 9, or the antibody-drug conjugate of claim 11 or the pharmaceutical composition of claim 12;
wherein preferably, the kit further comprises (i) a device for administering the antibody or the variant thereof, or the chimeric antigen receptor or the immune cell or the antibody-drug conjugate or the pharmaceutical composition; and/or (ii) instructions for use.

15. A kit of parts, **characterized by** comprising kit A and kit B, wherein
the kit A contains the antibody targeting CD25 or the antigen-binding fragment thereof of any one of claims 1-4, the chimeric antigen receptor of claim 5, the isolated nucleic acid of claim 6, the recombinant expression vector of claim 7, the transformant of claim 8, the immune cell of claim 9, the antibody-drug conjugate of claim 11 and/or the pharmaceutical composition of claim 12;
the kit B contains other anti-tumor antibodies or a pharmaceutical composition comprising the other anti-tumor antibodies, and/or one or more of the groups consisting of a hormone preparation, a targeting small molecule preparation, a proteasome inhibitor, an imaging agent, a diagnostic agent, a chemotherapeutic agent, an oncolytic drug, a cytotoxic agent, a cytokine, an activator of a costimulatory molecule, an inhibitor of an inhibitory molecule, and a vaccine.

16. A method for detecting CD25, **characterized in that** the method comprises using the antibody or the variant thereof of any one of claims 1-4, the chimeric antigen receptor of claim 5, the immune cell of claim 9, the antibody-drug conjugate of claim 11 and/or the pharmaceutical composition of claim 12; preferably, the detecting is for non-diagnostic and/or therapeutic purposes.

17. A method for preventing, treating or alleviating a cancer in a subject, **characterized in that** the method comprises administering to the subject a therapeutically effective amount of the antibody targeting CD25 or the variant thereof of any one of claims 1-4, the chimeric antigen receptor of claim 5, the isolated nucleic acid of claim 6, the recombinant expression vector of claim 7, the transformant of claim 8, the immune cell of claim 9, the antibody-drug conjugate of claim 11 and/or the pharmaceutical composition of claim 12.

18. A method for reducing the number of intra-tumor or tumor-infiltrating Treg cells in a subject, **characterized in that** the method comprises administering to the subject an effective amount of the antibody targeting CD25 or the variant thereof of any one of claims 1-4, the chimeric antigen receptor of claim 5, the isolated nucleic acid of claim 6, the recombinant expression vector of claim 7, the transformant of claim 8, the immune cell of claim 9, the antibody-drug conjugate of claim 11 and/or the pharmaceutical composition of claim 12.
